# EUROPEAN PATENT APPLICATION

(11) **EP 1 484 398 A1**
(43) Date of publication of application: **08.12.2004**
(21) Application number: 02788685.2
(22) Date of filing: 29.11.2002
(51) Int. Cl.: C12N 15/12, C12N 1/21, C12N 5/10, C07K 14/47, C07K 16/18, C07K 19/00, C12Q 1/02, C12Q 1/68, A01K 67/027, A61K 39/395, A61P 35/00

(54) **HUMAN GLIOMA ANTIGEN ORIGINATING IN TESTIS**

(30) Priority: 30.11.2001 JP 2001367960
(71) Applicant: Institute of Gene and Brain Science, Tokyo 160-0015 (JP)
(72) Inventor: TODA, Masahiro, Keio University,Sch.of Medicine, Tokyo 160-8582 (JP); KAWAKAMI, Yutaka, Keio University,Sch.of Medicine, Tokyo 160-8582 (JP); UEDA, Ryo, c/o Keio University, School of Medicine, Tokyo 160-8582 (JP)
(74) Representative: Harding, Charles Thomas
(86) International application number: PCT/JP2002/012502
(87) International publication number: WO 2003/046182

(57) **Abstract**

The present invention provides a testis-derived antigenic protein having an immunity induction activity comparable to a glioma antigenic protein and a gene encoding the same, etc., which can be applied to diagnosis and treatment of glioma. A testis-derived antigenic protein having an immunity induction activity comparable to a glioma antigenic protein and a gene encoding the same which can be applied to diagnosis and treatment of glioma are prepared by the steps of: extracting total RNA from testis, synthesizing cDNA, and constructing a λ phage cDNA library by introducing the cDNA into a λ phage vector and infecting Escherichia coli with the vector; reacting thus constructed λ phage cDNA library with the serum of a glioma patient, and using a labeled anti-IgG antibody to detect positive clones that react with the antibody in the serum; repeating the screening process several times on the detected positive clones to confirm the antibody reactivity; and isolating an antigen from the confirmed positive clones and performing serum screening using the isolated antigen and a glioma patient serum and a serum of a healthy individual, etc.

SEQUENCE LISTING

<110> Institute of Gene and Brain Science

<120> Human glioma antigen originating in testis

<130> P021134EP CTH

<140>
<141>

<150> JP P2001-367960
<151> 2001-11-30

<160> 20

<170> PatentIn Ver. 2.1

<210> 1
<211> 2898
<212> DNA
<213> Homo sapiens

<220>
<221> CDS
<222> (251)..(2848)

<400> 1
actttgttcg ctcctcagtc gtccaggcgg attccttttt cgccaggcac caaggcacag
60

cttagagtag acccgagtcc tgctctgcgg agttcgtctt cccagcgaag gtacagaggc
120

ggatgaactg ctgagacttg attgacgtat tttaagattt ttttaacttc tgaagtctag
180

caggcctgta agaacaaaaa tcattctgta ggaattaaaa acagaatcca gtcttgacaa
240

catatccaca atg tct gat gta tct act agt gta caa tca aaa ttt gct
289
           Met Ser Asp Val Ser Thr Ser Val Gln Ser Lys Phe Ala
             1               5                  10

aga ctt gca aag aaa aag gaa aat atc acc tat atg aaa aga gag cag
337
Arg Leu Ala Lys Lys Lys Glu Asn Ile Thr Tyr Met Lys Arg Glu Gln
     15                  20                  25

tta aca gaa act gat aag gac ata gct ccg gta tta gat tta aaa tgc

385
Leu Thr Glu Thr Asp Lys Asp Ile Ala Pro Val Leu Asp Leu Lys Cys
30                  35                  40                  45

aag gac gta tca gca att atg aat aag ttt aag gtc tta atg gaa att
433
Lys Asp Val Ser Ala Ile Met Asn Lys Phe Lys Val Leu Met Glu Ile
                50                  55                  60

caa gac ctg atg ttt gag gag atg agg gaa act ctt aaa aat gac cta
481
Gln Asp Leu Met Phe Glu Glu Met Arg Glu Thr Leu Lys Asn Asp Leu
            65                  70                  75

aaa gca gtt tta gga gga aaa gct aca ata cct gag gta aag aat tca
529
Lys Ala Val Leu Gly Gly Lys Ala Thr Ile Pro Glu Val Lys Asn Ser
        80                  85                  90

gag aac tcc agt agt agg aca gag ttt cag caa ata atc aat tta gca
577
Glu Asn Ser Ser Ser Arg Thr Glu Phe Gln Gln Ile Ile Asn Leu Ala
    95                  100                 105

tta caa aaa aca ggg atg gta ggg aaa ata gaa gga gaa aac tct aaa
625
Leu Gln Lys Thr Gly Met Val Gly Lys Ile Glu Gly Glu Asn Ser Lys
110                 115                 120                 125

ata ggt gat gat aat gaa aat tta acc ttt aaa tta gaa gta aat gag
673
Ile Gly Asp Asp Asn Glu Asn Leu Thr Phe Lys Leu Glu Val Asn Glu
                130                 135                 140

ctg agt ggt aaa tta gac aac act aac gaa tac aat agt aat gat ggt
721
Leu Ser Gly Lys Leu Asp Asn Thr Asn Glu Tyr Asn Ser Asn Asp Gly
            145                 150                 155

aag aaa tta ccc cag ggt gaa tca cga agt tac gaa gtc atg gga agt
769
Lys Lys Leu Pro Gln Gly Glu Ser Arg Ser Tyr Glu Val Met Gly Ser
        160                 165                 170

atg gaa gaa acc tta tgc aat ata gat gac aga gat gga aat cgc aat
817
Met Glu Glu Thr Leu Cys Asn Ile Asp Asp Arg Asp Gly Asn Arg Asn
    175                 180                 185

gtc cat tta gaa ttt aca gaa aga gag agt agg aag gat gga gag gat
865
Val His Leu Glu Phe Thr Glu Arg Glu Ser Arg Lys Asp Gly Glu Asp

190                 195                 200                 205

gaa ttt gtc aaa gaa atg aga gag gaa aga aaa ttt cag aaa ttg aag
913
Glu Phe Val Lys Glu Met Arg Glu Glu Arg Lys Phe Gln Lys Leu Lys
                210                 215                 220

aat aaa gag gag gtt tta aaa gcc tcc aga gaa gaa aaa gtg ttg atg
961
Asn Lys Glu Glu Val Leu Lys Ala Ser Arg Glu Glu Lys Val Leu Met
            225                 230                 235

gat gaa gga gca gta ctt acc ctg gta gcc gac ctt tca tca gca aca
1009
Asp Glu Gly Ala Val Leu Thr Leu Val Ala Asp Leu Ser Ser Ala Thr
        240                 245                 250

ctg gat att agt aag caa tgg agt aat gtc ttc aac att ctg aga gaa
1057
Leu Asp Ile Ser Lys Gln Trp Ser Asn Val Phe Asn Ile Leu Arg Glu
    255                 260                 265

aat gat ttt gaa cct aaa ttt ctg tgt gaa gtt aaa tta gca ttt aaa
1105
Asn Asp Phe Glu Pro Lys Phe Leu Cys Glu Val Lys Leu Ala Phe Lys
270                 275                 280                 285

tgt gat ggt gaa ata aag aca ttt tca gat ctg caa agc ctt aga aaa
1153
Cys Asp Gly Glu Ile Lys Thr Phe Ser Asp Leu Gln Ser Leu Arg Lys
                290                 295                 300

ttt gcc agc caa aaa tct tct gtg aaa gaa tta ctg aaa gat gta ctc
1201
Phe Ala Ser Gln Lys Ser Ser Val Lys Glu Leu Leu Lys Asp Val Leu
            305                 310                 315

cca caa aag gaa gaa ata aat caa gga gga aga aaa tat gga att caa
1249
Pro Gln Lys Glu Glu Ile Asn Gln Gly Gly Arg Lys Tyr Gly Ile Gln
        320                 325                 330

gaa aaa agg gat aaa acc cta ata gac tca aag cat aga gct gga gaa
1297
Glu Lys Arg Asp Lys Thr Leu Ile Asp Ser Lys His Arg Ala Gly Glu
    335                 340                 345

ata acc agt gat ggc ttg agc ttc cta ttt ctt aaa gaa gta aaa gtt
1345
Ile Thr Ser Asp Gly Leu Ser Phe Leu Phe Leu Lys Glu Val Lys Val
350                 355                 360                 365

gct aag cca gag gag atg aaa aac tta gag act caa gag gaa gag ttt
1393
Ala Lys Pro Glu Glu Met Lys Asn Leu Glu Thr Gln Glu Glu Glu Phe
                370                 375                 380

tcc gag cta gag gag ctg gat gaa gag gcc tca ggg atg gag gat gat
1441
Ser Glu Leu Glu Glu Leu Asp Glu Glu Ala Ser Gly Met Glu Asp Asp
            385                 390                 395

gaa gat acc tca ggg ctg gag gag gag gag gaa gag ccc tca ggg ctg
1489
Glu Asp Thr Ser Gly Leu Glu Glu Glu Glu Glu Glu Pro Ser Gly Leu
        400                 405                 410

gag gag gaa gaa gaa gaa gag gct tca ggg ttg gag gag gat gag gcc
1537
Glu Glu Glu Glu Glu Glu Glu Ala Ser Gly Leu Glu Glu Asp Glu Ala
    415                 420                 425

tca ggg cta gag gag gaa gag gaa cag act tca gaa cag gac tca acc
1585
Ser Gly Leu Glu Glu Glu Glu Glu Gln Thr Ser Glu Gln Asp Ser Thr
430                 435                 440                 445

ttt cag ggt cat act ttg gta gat gca aag cat gaa gtt gag ata acc
1633
Phe Gln Gly His Thr Leu Val Asp Ala Lys His Glu Val Glu Ile Thr
                450                 455                 460

agt gat ggc atg gaa act act ttc att gac tct gta gag gat tct gaa
1681
Ser Asp Gly Met Glu Thr Thr Phe Ile Asp Ser Val Glu Asp Ser Glu
            465                 470                 475

tca gag gag gaa gaa gaa gga aag agc tct gaa aca gga aag gta aag
1729
Ser Glu Glu Glu Glu Glu Gly Lys Ser Ser Glu Thr Gly Lys Val Lys
        480                 485                 490

act acc tcc ctg act gag aaa aaa gcc tca cgt aga caa aag gaa att
1777
Thr Thr Ser Leu Thr Glu Lys Lys Ala Ser Arg Arg Gln Lys Glu Ile
    495                 500                 505

ccc ttt agt tat ttg gtt ggg gac tct ggg aag aaa aag ttg gtg aaa
1825
Pro Phe Ser Tyr Leu Val Gly Asp Ser Gly Lys Lys Lys Leu Val Lys
510                 515                 520                 525

cac cag gtg gtg cac aaa acc cag gag gaa gag gaa aca gct gtg ccc
1873

His Gln Val Val His Lys Thr Gln Glu Glu Glu Thr Ala Val Pro
                530                 535                 540

aca agt caa gga act ggc ata ccc tgt ctg acc tta tgt ttg gcc tct
1921
Thr Ser Gln Gly Thr Gly Ile Pro Cys Leu Thr Leu Cys Leu Ala Ser
            545                 550                 555

ccc tca aag tca cta gag atg agt cat gat gag cat aaa aag cat tca
1969
Pro Ser Lys Ser Leu Glu Met Ser His Asp Glu His Lys Lys His Ser
        560                 565                 570

cat aca aat ttg agt att tca aca gga gtc acc aaa ctt aag aaa aca
2017
His Thr Asn Leu Ser Ile Ser Thr Gly Val Thr Lys Leu Lys Lys Thr
    575                 580                 585

gaa gaa aag aaa cac aga act ctg cac aca gaa gaa cta aca tcc aaa
2065
Glu Glu Lys Lys His Arg Thr Leu His Thr Glu Glu Leu Thr Ser Lys
590                 595                 600                 605

gaa gca gac tta aca gag gaa aca gaa gaa aac ttg aga agt agt gtg
2113
Glu Ala Asp Leu Thr Glu Glu Thr Glu Glu Asn Leu Arg Ser Ser Val
                610                 615                 620

att aat agc atc aga gag ata aaa gag gag att gga aat ttg aaa agt
2161
Ile Asn Ser Ile Arg Glu Ile Lys Glu Glu Ile Gly Asn Leu Lys Ser
            625                 630                 635

tcc cat tca ggt gtc ttg gaa att gaa aat tca gta gat gat ctg agt
2209
Ser His Ser Gly Val Leu Glu Ile Glu Asn Ser Val Asp Asp Leu Ser
        640                 645                 650

agc aga atg gac ata ctt gaa gaa aga ata gac agt cta gaa gat caa
2257
Ser Arg Met Asp Ile Leu Glu Glu Arg Ile Asp Ser Leu Glu Asp Gln
    655                 660                 665

att gaa gaa ttc tct aag gat aca atg caa atg acc aaa cag ata att
2305
Ile Glu Glu Phe Ser Lys Asp Thr Met Gln Met Thr Lys Gln Ile Ile
670                 675                 680                 685

agt aaa gaa agg caa aga gat ata gag gag aga tct aga agt tgc aac
2353
Ser Lys Glu Arg Gln Arg Asp Ile Glu Glu Arg Ser Arg Ser Cys Asn
                690                 695                 700

att cgt ttg ata gga att cca gaa aag gag agt tat gag aat agg gca
2401
Ile Arg Leu Ile Gly Ile Pro Glu Lys Glu Ser Tyr Glu Asn Arg Ala
                705                 710                 715

gag gac ata att aaa gaa ata att gat gaa aac ttt gca gaa cta aag
2449
Glu Asp Ile Ile Lys Glu Ile Ile Asp Glu Asn Phe Ala Glu Leu Lys
            720                 725                 730

aaa ggt tca agt ctt gag att gtc agt gct tgt cga gta cct agt aaa
2497
Lys Gly Ser Ser Leu Glu Ile Val Ser Ala Cys Arg Val Pro Ser Lys
        735                 740                 745

att gat gaa aag aga ctg act cct aga cac atc ttg gtg aaa ttt tgg
2545
Ile Asp Glu Lys Arg Leu Thr Pro Arg His Ile Leu Val Lys Phe Trp
750 755 760 765

aat tct agt gat aaa gag aaa ata ata agg gct tct aga gag aga aga
2593
Asn Ser Ser Asp Lys Glu Lys Ile Ile Arg Ala Ser Arg Glu Arg Arg
770 775 780

gaa att acc tac caa gga aca aga atc agg ttg aca gca gac tta tca
2641
Glu Ile Thr Tyr Gln Gly Thr Arg Ile Arg Leu Thr Ala Asp Leu Ser
                785                 790                 795

ctg gac aca ctg gat gct aga agt aaa tgg agc aat gtc ttc aaa gtt
2689
Leu Asp Thr Leu Asp Ala Arg Ser Lys Trp Ser Asn Val Phe Lys Val
            800                 805                 810

ctg ctg gaa aaa ggc ttt aat cct aga atc cta tat cca gcc aaa atg
2737
Leu Leu Glu Lys Gly Phe Asn Pro Arg Ile Leu Tyr Pro Ala Lys Met
        815                 820                 825

gca ttt gat ttt agg ggc aaa aca aag gta ttt ctt agt att gaa gaa
2785
Ala Phe Asp Phe Arg Gly Lys Thr Lys Val Phe Leu Ser Ile Glu Glu
830 835 840 845

ttt aga gat tat gtt ttg cat atg ccc acc ttg aga gaa tta ctg ggg
2833
Phe Arg Asp Tyr Val Leu His Met Pro Thr Leu Arg Glu Leu Leu Gly
850 855 860

aat aat ata cct tag cacgccaggg tgactacaaa caatatgctt tcctcccca

2888
Asn Asn Ile Pro
            865

gcatgcatcc
2898



<210> 2
<211> 865
<212> PRT
<213> Homo sapiens

<400> 2
Met Ser Asp Val Ser Thr Ser Val Gln Ser Lys Phe Ala Arg Leu Ala
1               5                   10                  15
Lys Lys Lys Glu Asn Ile Thr Tyr Met Lys Arg Glu Gln Leu Thr Glu
            20                  25                  30
Thr Asp Lys Asp Ile Ala Pro Val Leu Asp Leu Lys Cys Lys Asp Val
        35                  40                  45
Ser Ala Ile Met Asn Lys Phe Lys Val Leu Met Glu Ile Gln Asp Leu
    50                  55                  60
Met Phe Glu Glu Met Arg Glu Thr Leu Lys Asn Asp Leu Lys Ala Val
65                  70                  75                  80
Leu Gly Gly Lys Ala Thr Ile Pro Glu Val Lys Asn Ser Glu Asn Ser
                85                  90                  95
Ser Ser Arg Thr Glu Phe Gln Gln Ile Ile Asn Leu Ala Leu Gln Lys
            100                 105                 110
Thr Gly Met Val Gly Lys Ile Glu Gly Glu Asn Ser Lys Ile Gly Asp
        115                 120                 125
Asp Asn Glu Asn Leu Thr Phe Lys Leu Glu Val Asn Glu Leu Ser Gly
    130                 135                 140
Lys Leu Asp Asn Thr Asn Glu Tyr Asn Ser Asn Asp Gly Lys Lys Leu
145                 150                 155                 160
Pro Gln Gly Glu Ser Arg Ser Tyr Glu Val Met Gly Ser Met Glu Glu
                165                 170                 175
Thr Leu Cys Asn Ile Asp Asp Arg Asp Gly Asn Arg Asn Val His Leu
            180                 185                 190
Glu Phe Thr Glu Arg Glu Ser Arg Lys Asp Gly Glu Asp Glu Phe Val
        195                 200                 205
Lys Glu Met Arg Glu Glu Arg Lys Phe Gln Lys Leu Lys Asn Lys Glu
    210                 215                 220
Glu Val Leu Lys Ala Ser Arg Glu Glu Lys Val Leu Met Asp Glu Gly
225                 230                 235                 240
Ala Val Leu Thr Leu Val Ala Asp Leu Ser Ser Ala Thr Leu Asp Ile
                245                 250                 255
Ser Lys Gln Trp Ser Asn Val Phe Asn Ile Leu Arg Glu Asn Asp Phe
            260                 265                 270
Glu Pro Lys Phe Leu Cys Glu Val Lys Leu Ala Phe Lys Cys Asp Gly
        275                 280                 285
Glu Ile Lys Thr Phe Ser Asp Leu Gln Ser Leu Arg Lys Phe Ala Ser
    290                 295                 300

Gln Lys Ser Ser Val Lys Glu Leu Leu Lys Asp Val Leu Pro Gln Lys
305                 310                 315                 320
Glu Glu Ile Asn Gln Gly Gly Arg Lys Tyr Gly Ile Gln Glu Lys Arg
                325                 330                 335
Asp Lys Thr Leu Ile Asp Ser Lys His Arg Ala Gly Glu Ile Thr Ser
            340                 345                 350
Asp Gly Leu Ser Phe Leu Phe Leu Lys Glu Val Lys Val Ala Lys Pro
        355                 360                 365
Glu Glu Met Lys Asn Leu Glu Thr Gln Glu Glu Glu Phe Ser Glu Leu
    370                 375                 380
Glu Glu Leu Asp Glu Glu Ala Ser Gly Met Glu Asp Asp Glu Asp Thr
385                 390                 395                 400
Ser Gly Leu Glu Glu Glu Glu Glu Glu Pro Ser Gly Leu Glu Glu Glu
                405                 410                 415
Glu Glu Glu Glu Ala Ser Gly Leu Glu Glu Asp Glu Ala Ser Gly Leu
            420                 425                 430
Glu Glu Glu Glu Glu Gln Thr Ser Glu Gln Asp Ser Thr Phe Gln Gly
        435                 440                 445
His Thr Leu Val Asp Ala Lys His Glu Val Glu Ile Thr Ser Asp Gly
    450                 455                 460
Met Glu Thr Thr Phe Ile Asp Ser Val Glu Asp Ser Glu Ser Glu Glu
465                 470                 475                 480
Glu Glu Glu Gly Lys Ser Ser Glu Thr Gly Lys Val Lys Thr Thr Ser
                485                 490                 495
Leu Thr Glu Lys Lys Ala Ser Arg Arg Gln Lys Glu Ile Pro Phe Ser
            500                 505                 510
Tyr Leu Val Gly Asp Ser Gly Lys Lys Lys Leu Val Lys His Gln Val
        515                 520                 525
Val His Lys Thr Gln Glu Glu Glu Glu Thr Ala Val Pro Thr Ser Gln
    530                 535                 540
Gly Thr Gly Ile Pro Cys Leu Thr Leu Cys Leu Ala Ser Pro Ser Lys
545                 550                 555                 560
Ser Leu Glu Met Ser His Asp Glu His Lys Lys His Ser His Thr Asn
                565                 570                 575
Leu Ser Ile Ser Thr Gly Val Thr Lys Leu Lys Lys Thr Glu Glu Lys
            580                 585                 590
Lys His Arg Thr Leu His Thr Glu Glu Leu Thr Ser Lys Glu Ala Asp
        595                 600                 605
Leu Thr Glu Glu Thr Glu Glu Asn Leu Arg Ser Ser Val Ile Asn Ser
    610                 615                 620
Ile Arg Glu Ile Lys Glu Glu Ile Gly Asn Leu Lys Ser Ser His Ser
625                 630                 635                 640
Gly Val Leu Glu Ile Glu Asn Ser Val Asp Asp Leu Ser Ser Arg Met
                645                 650                 655
Asp Ile Leu Glu Glu Arg Ile Asp Ser Leu Glu Asp Gln Ile Glu Glu
            660                 665                 670
Phe Ser Lys Asp Thr Met Gln Met Thr Lys Gln Ile Ile Ser Lys Glu
        675                 680                 685
Arg Gln Arg Asp Ile Glu Glu Arg Ser Arg Ser Cys Asn Ile Arg Leu
    690                 695                 700
Ile Gly Ile Pro Glu Lys Glu Ser Tyr Glu Asn Arg Ala Glu Asp Ile
705                 710                 715                 720

Ile Lys Glu Ile Ile Asp Glu Asn Phe Ala Glu Leu Lys Lys Gly Ser
                725                 730                 735
Ser Leu Glu Ile Val Ser Ala Cys Arg Val Pro Ser Lys Ile Asp Glu
            740                 745                 750
Lys Arg Leu Thr Pro Arg His Ile Leu Val Lys Phe Trp Asn Ser Ser
        755                 760                 765
Asp Lys Glu Lys Ile Ile Arg Ala Ser Arg Glu Arg Arg Glu Ile Thr
    770                 775                 780
Tyr Gln Gly Thr Arg Ile Arg Leu Thr Ala Asp Leu Ser Leu Asp Thr
785                 790                 795                 800
Leu Asp Ala Arg Ser Lys Trp Ser Asn Val Phe Lys Val Leu Leu Glu
                805                 810                 815
Lys Gly Phe Asn Pro Arg Ile Leu Tyr Pro Ala Lys Met Ala Phe Asp
            820                 825                 830
Phe Arg Gly Lys Thr Lys Val Phe Leu Ser Ile Glu Glu Phe Arg Asp
        835                 840                 845
Tyr Val Leu His Met Pro Thr Leu Arg Glu Leu Leu Gly Asn Asn Ile
    850                 855                 860
Pro
865

<210> 3
<211> 1748
<212> DNA
<213> Homo sapiens

<220>
<221> CDS
<222> (421)..(1617)

<400> 3
gaagcgggtc ccgcaggtcg ccacggttgg gggaaacgcg gcggacgccg cccccgtccc
60

gaaggggact cgaaaatgta cagccagcgg tttggcaccg tacagcggga ggttaagggc
120

cccaccccca aagtggtgat cgtgagatcc aagcctccta aaggccaagg agctgagcac
180

catctagaaa gaatccgacg cagccatcag aagcataatg ctattttggc ttccattaag
240

tcaagtgagc gggatcgctt gaaagctgag tgggaccagc acaatgactg caagattttg
300

gacagccttg tgcgagcaag aatcaaggat gctgtgcaag ggtttatcat taacattgaa
360

gaaagacgaa ataagctacg tgagctttta gcattagaag aaaatgagta ttttacagaa

420

atg caa ttg aag aaa gaa acc att gag gag aaa aaa gat agg atg aga
468
Met Gln Leu Lys Lys Glu Thr Ile Glu Glu Lys Lys Asp Arg Met Arg
1               5                   10                  15

gag aaa act aaa tta cta aaa gag aag aat gaa aaa gag agg cag gat
516
Glu Lys Thr Lys Leu Leu Lys Glu Lys Asn Glu Lys Glu Arg Gln Asp
            20                  25                  30

ttt gtg gct gaa aag cta gac cag caa ttc agg gaa cgc tgt gag gag
564
Phe Val Ala Glu Lys Leu Asp Gln Gln Phe Arg Glu Arg Cys Glu Glu
        35                  40                  45

ctc cgt gtt gaa ttg tta tct atc cat cag aag aag gtg tgt gag gag
612
Leu Arg Val Glu Leu Leu Ser Ile His Gln Lys Lys Val Cys Glu Glu
    50                  55                  60

cgg aaa gca cag att gca ttt aat gag gag ctg agc agg caa aag ctg
660
Arg Lys Ala Gln Ile Ala Phe Asn Glu Glu Leu Ser Arg Gln Lys Leu
65                  70                  75                  80

gtg gaa gag cag atg ttc tcc aaa ctc tgg gag gaa gac cga tta gcc
708
Val Glu Glu Gln Met Phe Ser Lys Leu Trp Glu Glu Asp Arg Leu Ala
                85                  90                  95

aag gaa aag cga gaa gcc caa gag gcg agg aga cag aaa gag ctg atg
756
Lys Glu Lys Arg Glu Ala Gln Glu Ala Arg Arg Gln Lys Glu Leu Met
            100                 105                 110

gag aac aca cgc ctg ggg ctg aat gcc cag atc acc agc atc aag gca
804
Glu Asn Thr Arg Leu Gly Leu Asn Ala Gln Ile Thr Ser Ile Lys Ala
        115                 120                 125

caa agg cag gcg aca cag ctg ctg aag gaa gag gag gca cgc ctt gtg
852
Gln Arg Gln Ala Thr Gln Leu Leu Lys Glu Glu Glu Ala Arg Leu Val
    130                 135                 140

gaa agt aac aac gca cag att aaa cat gag aat gaa cag gat atg cta
900
Glu Ser Asn Asn Ala Gln Ile Lys His Glu Asn Glu Gln Asp Met Leu
145                 150                 155                 160

aag aaa cag aag gca aag cag gaa act agg acc att ttg caa aaa gcc
948
Lys Lys Gln Lys Ala Lys Gln Glu Thr Arg Thr Ile Leu Gln Lys Ala
                165                 170                 175

cta caa gag agg ata gaa cat att cag cag gaa tac aga gac gaa cag
996
Leu Gln Glu Arg Ile Glu His Ile Gln Gln Glu Tyr Arg Asp Glu Gln
            180                 185                 190

gac ttg aac atg aag ctc gtg caa agg gcc ctt caa gac tta cag gaa
1044
Asp Leu Asn Met Lys Leu Val Gln Arg Ala Leu Gln Asp Leu Gln Glu
        195                 200                 205

gag gca gat aaa aag aaa caa aaa aga gaa gat atg ata aga gaa cag
1092
Glu Ala Asp Lys Lys Lys Gln Lys Arg Glu Asp Met Ile Arg Glu Gln
    210                 215                 220

aag ata tac cat aaa tat ttg gca cag aga cgt gag gaa gaa aaa gct
1140
Lys Ile Tyr His Lys Tyr Leu Ala Gln Arg Arg Glu Glu Glu Lys Ala
225                 230                 235                 240

cag gag aaa gaa ttt gac aga ata tta gag gaa gac aag gca aag aag
1188
Gln Glu Lys Glu Phe Asp Arg Ile Leu Glu Glu Asp Lys Ala Lys Lys
                245                 250                 255

ttg gct gag aag gac aag gag ctg aga ctt gaa aag gag gca agg aga
1236
Leu Ala Glu Lys Asp Lys Glu Leu Arg Leu Glu Lys Glu Ala Arg Arg
            260                 265                 270

cag ctt gtg gat gag gtc atg tgt aca aga aaa ctt caa gtt caa gaa
1284
Gln Leu Val Asp Glu Val Met Cys Thr Arg Lys Leu Gln Val Gln Glu
        275                 280                 285

aag ttg caa cga gaa gct aaa gaa cag gaa gaa cgt gct atg gaa cag
1332
Lys Leu Gln Arg Glu Ala Lys Glu Gln Glu Glu Arg Ala Met Glu Gln
    290                 295                 300

aaa cac ata aat gaa agt ctt aaa gaa ctt aac tgt gaa gag aag gag
1380
Lys His Ile Asn Glu Ser Leu Lys Glu Leu Asn Cys Glu Glu Lys Glu
305                 310                 315                 320

aat ttt gca aga cgc caa cgt tta gcc cag gag tac agg aag caa ctt
1428

Asn Phe Ala Arg Arg Gln Arg Leu Ala Gln Glu Tyr Arg Lys Gln Leu
                325                 330                 335

cag atg caa atc gcc tac cag cag cag tcc caa gaa gca gag aag gaa
1476
Gln Met Gln Ile Ala Tyr Gln Gln Gln Ser Gln Glu Ala Glu Lys Glu
            340                 345                 350

gag aaa cgc cga gag ttt gaa gca ggt gta gca gca aac aag atg tgt
1524
Glu Lys Arg Arg Glu Phe Glu Ala Gly Val Ala Ala Asn Lys Met Cys
        355                 360                 365

ttg gac aag gtc cag gag gtc ctg tcc acc cat caa gtg ctg cct caa
1572
Leu Asp Lys Val Gln Glu Val Leu Ser Thr His Gln Val Leu Pro Gln
    370                 375                 380

aac att cat ccc atg cgc aag gca tgc ccc agt aag ctt cca ccg
1617
Asn Ile His Pro Met Arg Lys Ala Cys Pro Ser Lys Leu Pro Pro
385                 390                 395

tagttccgtg agcatcaata tatcttttct tggtctttta atatttttaa ctacagtatg
1677

cttgtatgct tcttttaact cctggataaa cttttctttt ttccctgaaa aaaaaaaaaa
1737

aaaaaaaaaa a
1748



<210> 4
<211> 399
<212> PRT
<213> Homo sapiens

<400> 4
Met Gln Leu Lys Lys Glu Thr Ile Glu Glu Lys Lys Asp Arg Met Arg
  1               5                  10                  15

Glu Lys Thr Lys Leu Leu Lys Glu Lys Asn Glu Lys Glu Arg Gln Asp
             20                  25                  30

Phe Val Ala Glu Lys Leu Asp Gln Gln Phe Arg Glu Arg Cys Glu Glu
         35                  40                  45

Leu Arg Val Glu Leu Leu Ser Ile His Gln Lys Lys Val Cys Glu Glu
     50                  55                  60

Arg Lys Ala Gln Ile Ala Phe Asn Glu Glu Leu Ser Arg Gln Lys Leu

65                  70                  75                  80

Val Glu Glu Gln Met Phe Ser Lys Leu Trp Glu Glu Asp Arg Leu Ala
                85                  90                  95

Lys Glu Lys Arg Glu Ala Gln Glu Ala Arg Arg Gln Lys Glu Leu Met
            100                 105                 110

Glu Asn Thr Arg Leu Gly Leu Asn Ala Gln Ile Thr Ser Ile Lys Ala
        115                 120                 125

Gln Arg Gln Ala Thr Gln Leu Leu Lys Glu Glu Glu Ala Arg Leu Val
    130                 135                 140

Glu Ser Asn Asn Ala Gln Ile Lys His Glu Asn Glu Gln Asp Met Leu
145                 150                 155                 160

Lys Lys Gln Lys Ala Lys Gln Glu Thr Arg Thr Ile Leu Gln Lys Ala
                165                 170                 175

Leu Gln Glu Arg Ile Glu His Ile Gln Gln Glu Tyr Arg Asp Glu Gln
            180                 185                 190

Asp Leu Asn Met Lys Leu Val Gln Arg Ala Leu Gln Asp Leu Gln Glu
        195                 200                 205

Glu Ala Asp Lys Lys Lys Gln Lys Arg Glu Asp Met Ile Arg Glu Gln
    210                 215                 220

Lys Ile Tyr His Lys Tyr Leu Ala Gln Arg Arg Glu Glu Glu Lys Ala
225                 230                 235                 240

Gln Glu Lys Glu Phe Asp Arg Ile Leu Glu Glu Asp Lys Ala Lys Lys
                245                 250                 255

Leu Ala Glu Lys Asp Lys Glu Leu Arg Leu Glu Lys Glu Ala Arg Arg
            260                 265                 270

Gln Leu Val Asp Glu Val Met Cys Thr Arg Lys Leu Gln Val Gln Glu
        275                 280                 285

Lys Leu Gln Arg Glu Ala Lys Glu Gln Glu Glu Arg Ala Met Glu Gln
    290                 295                 300

Lys His Ile Asn Glu Ser Leu Lys Glu Leu Asn Cys Glu Glu Lys Glu
305                 310                 315                 320

Asn Phe Ala Arg Arg Gln Arg Leu Ala Gln Glu Tyr Arg Lys Gln Leu
                325                 330                 335

Gln Met Gln Ile Ala Tyr Gln Gln Gln Ser Gln Glu Ala Glu Lys Glu
            340                 345                 350

Glu Lys Arg Arg Glu Phe Glu Ala Gly Val Ala Ala Asn Lys Met Cys
        355                 360                 365

Leu Asp Lys Val Gln Glu Val Leu Ser Thr His Gln Val Leu Pro Gln
    370                 375                 380

Asn Ile His Pro Met Arg Lys Ala Cys Pro Ser Lys Leu Pro Pro
385                 390                 395

<210> 5
<211> 20
<212> DNA
<213> Artificial Sequence

<220>
<223> Description of Artificial Sequence:T3

<400> 5
aattaaccct cactaaaggg
20

<210> 6
<211> 22
<212> DNA
<213> Artificial Sequence

<220>
<223> Description of Artificial Sequence:T7

<400> 6
gtaatacgac tcactatagg gc
22

<210> 7
<211> 24
<212> DNA
<213> Artificial Sequence

<220>
<223> Description of Artificial Sequence:KU-GB-3 Sense
      Primer (P1)

<400> 7
tcaaggaact ggcataccct gtct
24

<210> 8
<211> 24
<212> DNA
<213> Artificial Sequence

<220>
<223> Description of Artificial Sequence:KU-GB-3
      Antisense Primer(P2)

<400> 8
ggtactcgac aagcactgac aatc
24



<210> 9
<211> 20
<212> DNA
<213> Artificial Sequence

<220>
<223> Description of Artificial Sequence:KU-GB-4 Sense
      Primer(P3)

<400> 9
caaactctgg gaggaagacc
20



<210> 10
<211> 20
<212> DNA
<213> Artificial Sequence

<220>
<223> Description of Artificial Sequence:KU-GB-4
      Antisense Primer(P4)

<400> 10
agttggctga gaaggacaag
20



<210> 11
<211> 19
<212> DNA
<213> Artificial Sequence

<220>
<223> Description of Artificial Sequence:SOX5 Sense
      Primer(P5)

<400> 11

gcaacaggag gaaggaaat
19

<210> 12
<211> 22
<212> DNA
<213> Artificial Sequence

<220>
<223> Description of Artificial Sequence:SOX5 Antisense
      Primer(P6)

<400> 12
gacaagccaa ctgataaggg tc
22

<210> 13
<211> 22
<212> DNA
<213> Artificial Sequence

<220>
<223> Description of Artificial Sequence:SOX6 Sense
      Primer(P7)

<400> 13
cgcgctttga gaatttgggg cc
22

<210> 14
<211> 22
<212> DNA
<213> Artificial Sequence

<220>
<223> Description of Artificial Sequence:SOX6 Antisense
      Primer(P8)

<400> 14
tctttgttgg ggaggggggt ga
22

<210> 15
<211> 24
<212> DNA
<213> Artificial Sequence

<220>

<223> Description of Artificial Sequence:ZINC FINGER
      HOMEOBOX 1B Sense Primer(P9)

<400> 15
tggagatcac tccatggacg atag
24


<210> 16
<211> 23
<212> DNA
<213> Artificial Sequence

<220>
<223> Description of Artificial Sequence:ZINC FINGER
      HOMEOBOX 1B Antisense Primer(P10)

<400> 16
cctgttcttt gaagcaccca tgt
23


<210> 17
<211> 16
<212> DNA
<213> Artificial Sequence

<220>
<223> Description of Artificial Sequence:M-PHASE
      PHOSPHOPROTEIN1 Sense Primer(P11)

<400> 17
aacgagccaa acggaa
16


<210> 18
<211> 18
<212> DNA
<213> Artificial Sequence

<220>
<223> Description of Artificial Sequence:M-PHASE
      PHOSPHOPROTEIN1 Antisense Primer(P12)

<400> 18
gtatccatcc cctcaagc
18


<210> 19
<211> 25

<212> DNA
<213> Artificial Sequence

<220>
<223> Description of Artificial Sequence:beta-actin
      sense primer(P13)

<400> 19
gtcgacaacg gctccggcat gtgca
25


<210> 20
<211> 25
<212> DNA
<213> Artificial Sequence

<220>
<223> Description of Artificial Sequence:beta-actin
      antisense primer(P14)

<400> 20
ggatcttcat gaggtagtca gtcag
25

## Description

### Technical Field

The present invention relates to a testis-derived antigenic protein, having an immunity induction activity comparable to a glioma antigen, expressed and exhibiting an immunological response in glioma, which is a malignant brain tumor of humans. The present invention also relates to a gene encoding the abovementioned antigenic protein as well as an immunity induction promoter or suppressor screening method, a glioma detection/diagnosis probe, antitumor agent, etc., that use the glioma antigen and a gene encoding the same.

### Background Art

Though immunotherapy has been anticipated since previously and various efforts have been made, adequate antitumor effects have yet to be demonstrated. Though nonspecific immunotherapy methods have mainly been practiced for immunotherapy of cancers priorly, it has recently become known that with human melanoma, T cells play an important role in tumor rejection in a living body, and since the reporting of the MAGE-1 antigen by a Belgian group in 1991 (Science 254, 1643-7, 1991), efforts are being made to isolate T cell recognizing tumor antigens that can induce cytotoxic T lymphocytes (CTLs) and to determine the MHC class I binding epitope. The present inventors have isolated a CD8⁺ T cell recognizing antigen by cDNA expression cloning using tumor reactive T cells of melanoma (Proc. Natl. Acad. Sci. USA 91, 3515-3519, 1994; Proc. Natl. Acad. Sci. USA 91, 6458-6462, 1994; J. Exp. Med. 180, 347-352, 1994; J. Immunol. 154, 3961-3968, 1995; Immunologic Res. 16, 313-340, 1997) and have reported that specific immunotherapy using this antigen exhibits antitumor effects for some types of melanoma (Microbiology Immunology 42, 803-813, 1998; Kawakami, Y., P. F. Robbins, R. F. Wang et al. Identification of melanoma antigens by T lymphocytes and their use in the immunotherapy of cancer. In Principle and Practice of Oncology. Update. V. DeVita, S. Hellman, S. A. Rosenberg eds. J. B. Lippincott Co. Philadelphia, p1-20, 1996; Nature Med. 4, 321, 1998). It has also been found that an antigen that is recognized by T cells is a peptide that is bound to an MHC molecule, that the peptide does not have to originate in a cell surface protein, and an intranuclear protein or a cytoplasmic protein may also be recognized as an antigen (Immunity 10, 281-7, 1999).

In 1995, the group of Pfreundschuh of Germany and Old and others of the USA reported SEREX (serological identification of recombinant cDNA expression cloning; Proc. Natl. Acad. Sci. USA 92, 11810-11813, 1995) as a method of detecting cancer antigenic proteins, recognized by IgG antibodies in the sera of cancer patients, that requires neither tumors nor CTLs and can be applied to tumors for which the establishment of a cell line is difficult. Several tumor antigens have been isolated by this method, and since MAGE-1, tyrosinase, and other antigens that induce CTLs are included among the antigens isolated by this method, the method has been indicated as being useful for the detection of antigens that are recognized by cellular immunity. It has also been reported that the above method has enabled isolation of cancer antigens, recognized by patient IgG antibodies, in melanoma, renal cancer, esophageal cancer, colon cancer, lung cancer, etc., (Int. J. Cancer 72, 965-971, 1997; Cancer Res. 58, 1034-1041, 1998; Int. J. Cancer 29, 652-658, 1998; Int. J. Oncol. 14, 703-708, 1999; Cancer Res. 56, 4766-4772, 1996; Hum. Mol. Genet 6, 33-39, 1997). These antigens include CT (cancer-testis) antigens (SSX2/HOM-MEL-40, NY-ESO-1, SCP-1, CT7, etc.), differentiation antigens (galectin-4/NY-CO-27), mutation antigens (p53, etc.), and other important antigens. In particular among these antigens, the HLA-A2 binding epitope has been determined for NY-ESO-1. The antigens isolated by SEREX also include molecules, such as elF-4γ and HER2/neu, which are highly expressed in cancers and these molecules are possibly related to cancerization. Such molecules that are highly expressed in cancers may be applied not only to treatment but has possibilities as diagnostic markers or as markers for estimating recurrence or prognosis.

Meanwhile, the central nervous system has been known as an immune-privileged site and recent studies have shown that stimulated lymphocytes can enter the brain across the blood-brain barrier and react with brain antigens (J. Immunol. 158, 2318-26, 1997; J. Neurosci. 18, 5804-16, 1998). The present inventors have also suggested the possibility of specific immunity induction against tumor antigens in the brain as a new, effective treatment method (Neuro-Oncology 1, S015, 1999). Though presently, image analysis by MRI, etc., and histopathological analysis using biopsy samples are mainly carried out as methods of diagnosing gliomas, which are malignant brain tumors, the genes concerned with the occurrence of glioma are becoming clarified with the progress of molecular cell biology in recent years and the possibilities of genetic diagnosis, in which these genes are analyzed, have also been suggested (Glia. 15, 308-27, 1995).

The SOX gene family is a group of transcription factors defined by the SRY-related HMG box region, which is well conserved and mediates sequence-specific DNA binding (Curr. Opin. Genet. Dev. 7, 338-344, 1997; Nucleic Acids Res. 27, 1409-1420, 1999). With the discovery of the SRY gene (the testes-determining gene on the Y chromosome that determines the male sex) in 1990, a gene group, indicating high homology (>60%) with the SRY HMG box, has been identified by cross hybridization and PCR, and this gene group forms a new subfamily within the HMG box superfamily. SOX genes have been found in a wide range of species from vertebrates, including humans and mice, to invertebrates (fruit fly). At least 20 types of SOX genes have been reported to exist in mice.

The known functions of SOX proteins are diverse and complex. Sry is involved in sex determination (Nature 364, 713-715, 1993), SOX1-3 in lens development (Development 125, 2521-2532, 1998), SOX4 in endocardial ridge development in the heart andpro-B cell proliferation (Nature 380, 711-714, 1996), SOX9 in chondrogenesis and sex determination (Nat. Genet. 22, 85-89, 1999; Cell 79, 1111-1120, 1994) and SOX10 in neural crest cell differentiation (Nat. Genet. 18, 60-64, 1998; Nat. Genet. 18, 171-173, 1998). Such studies provide evidence that SOX genes serve a regulatory role in developmental pathways.

Like the behavior of other SOX genes, the function of the SOX6 protein is considered to be concerned with the restriction of embryonic development and the determination of cell destinies. The mouse SOX6 has been reported to be expressed in the central nervous system during embryogenesis, and though it is expressed until 12.5 days from mating, it has not been detected from the adult brain (Nucleic Acid Res. 23, 3365-3372, 1995). SOX6, SOX5, and SOX9 were found to be expressed at high levels in all cartilaginous parts of the mouse embryo from the early stages of chondrogenesis (Embo. J. 17, 5718-5733, 1998). According to prior reports (Embo. J. 17, 5718-5733, 1998; Gene 265, 157-164, 2001), though the mouse SOX6 is expressed in the fetal brain and primary chondrocytes, such expression is lower than the expression in testis.

Also, two major size classes of SOX6 transcripts have been reported for mice (Proc. Natl. Acad. Sci. U.S.A. 97, 4180-4185, 2000), rats (Biol. Pharm. Bull. 25, 705-709, 2002), and humans (Gene 265, 157-164, 2001). The shorter transcript is approximately 3 kb and the longer transcript is 8 to 9 kb. The difference of these transcripts has been reported to be due to the difference in the respective sizes of the 3' and 5' UTRs (untranslated regions) (Embo. J. 17, 5718-5733, 1998), though the possibility of the differences being due to different promoters and different RNA processing cannot be denied.

Presently, even though progresses have been made in the knowledge of the mechanisms of occurrence, diagnostic methods, and treatment methods of cancers, which have become the number one cause of death, much of the advanced cancers cannot be cured in actuality. In particular, malignant brain tumors are disorders that are extremely difficult to cure, and despite progresses in brain surgery and radiation therapy, there have been no effective treatments methods as of yet. The development of new early diagnostic methods and treatment methods is deemed necessary for improvement of the present situation. An object of the present invention is to provide a testis-derived antigenic protein, which has an immunity induction activity comparable to a glioma antigen and is applicable to the diagnosis and treatment of glioma, a gene encoding the same, etc.

In order to achieve the above object, the present inventors carried out diligent research and, by SEREX using a testis-derived cDNA library and glioma patient sera, obtained new testis-derived antigenic proteins, which react only with glioma patient sera, and new testis-derived antigenic proteins, which react with the sera of many glioma patients but hardly react with the sera of healthy individuals. By RT-PCR using glioma antigens, it has been found that the testis-derived antigenic proteins, having immunity induction activities comparable to the glioma antigens, and the DNAs encoding the same are useful for the diagnosis and treatment of glioma and have come to complete the present invention.

### Disclosure of the Invention

The present invention relates to a DNA encoding a protein comprising the amino acid sequence indicated by SEQ ID NO. 2 or 4, or a DNA encoding a protein comprising an amino acid sequence in which one or several amino acids have been deleted, substituted, or added with respect to the amino acid sequence indicated by SEQ ID NO. 2 or 4 and having an immunity induction activity ("1"), a DNA comprising the base sequence indicated by SEQ ID NO. 1 or 3 or a base sequence complementary to the same or a base sequence containing a part or the whole of the same ("2"), a DNA which hybridizes under stringent conditions with the DNA according to "2" and encodes a protein having an immunity induction activity ("3"), and an oligonucleotide or peptide nucleic acid which hybridizes specifically with the DNA according to "3" or with an RNA, corresponding to this DNA, and inhibits the expression of a protein having an immunity induction activity ("4").

The present invention also relates to a protein comprising the amino acid sequence indicated by SEQ ID NO. 2 or 4, or a protein comprising an amino acid sequence in which one or several amino acids have been deleted, substituted, or added with respect to the amino acid sequence indicated by SEQ ID NO. 2 or 4 and having an immunity induction activity ("5"), a peptide comprising a part of the protein according to "5" and having an immunity induction activity ("6"), a fusion protein or fusion peptide, wherein the protein according to "5" or the peptide according to "6" is bound with a marker protein and/or a peptide tag ("7"), an antibody against the protein according to "5" or the peptide according to "6" ("8"), a recombinant vector containing the DNA according to any of "1" to "4" ("9"), a host cell containing an expression system that can express the protein according to "5" or the peptide according to "6" ("10"), a non-human animal, in which a genetic function encoding the protein according to "5" or the peptide according to "6" is absent from the chromosomes or which overexpresses the protein according to "5" or the peptide according to "6" ("11"), and an immunity induction activity promoter or inhibitor screening method using the protein according to "5" or the peptide according to "6", a test substance, and T cells and measuring and evaluating the immunity induction activity in the T cells ("12").

The present invention also relates to a testis-derived antigenic protein having an immunity induction activity comparable to a glioma antigenic protein and being obtained by the steps of: (1) extracting and purifying total RNA from testis tissue, synthesizing cDNA using the purified mRNA, and constructing a λ phage cDNA library by introducing the cDNA into a λ phage vector and infecting Escherichia coli with the vector; (2) diluting the serum of a glioma patient, reacting it with an extract of the Escherichia coli, and removing the reaction products to prepare a reaction serum; (3) reacting the abovementioned λ phage cDNA library with the reaction serum and using a labeled anti-IgG antibody to detect positive clones that react with the antibody in the serum; (4) repeating the screening process several times on the detected positive clones to confirm the antibody reactivity; and (5) isolating an antigen from the positive clones and performing serum screening using the isolated antigen and at least a glioma patient serum and a serum of a healthy individual ("13"), the testis-derived antigenic protein according to "13", comprising KU-GB-4, KU-GB-3, a SOX family molecule, zinc finger homeobox (zinc finger homeobox 1B), an ADP-ribosylation factor (ADP-ribosylation factor 4-like), or M-phase phosphoprotein 1 ("14"), the testis-derived antigenic protein according to "14", wherein the SOX family molecule is SOX5, SOX6, or SOX13 ("15"), a glioma detection diagnostic drug containing the whole or a part of the protein according to any of "13" to "15" and/or an antibody binding specifically with the whole or a part of the protein according to any of "13" to "15" ("16"), a glioma diagnosis method characterized in using an antibody binding specifically with the whole or a part of the protein according to any of "13" to "15" ("17"), the glioma diagnosis method according to "17", wherein a test sample is a serum and ELISA or Western blotting is employed ("18"), the glioma diagnosis method according to "17", wherein a test sample is a cell tissue and an immunohistochemical analysis method is employed ("19"), a glioma detection/diagnosis probe containing the whole or a part of an antisense chain of a DNA or RNA encoding the protein according to any of "13" to "15" ("20"), an antitumor agent characterized in containing the whole or part of the protein according to any of "13" to "15" and/or an antibody binding specifically with the whole or part of the abovementioned glioma antigen as an effective component ("21"), and a tumor prevention/treatment drug using the antitumor agent according to "21" ("22").

The present invention furthermore relates to a glioma recognizing antibody specifically recognizing glioma in immunohistochemical analysis ("23"), the glioma recognizing antibody according to "23", which is an anti-SOX6 antibody ("24"), the glioma recognizing antibody according to "24", wherein the anti-SOX6 antibody specifically recognizes the HMG box of SOX6 ("25"), a glioma detection diagnostic drug containing the glioma recognizing antibody according to any of "23" to "25" ("26"), a glioma diagnosis method using the glioma recognizing antibody according to any of "23" to "25" ("27"), the glioma diagnosis method according to "27", wherein a test sample is a serum and ELISA or Western blotting is employed ("28"), the glioma diagnosis method according to "27", wherein a test sample is a cell tissue and an immunohistochemical analysis method is employed ("29"), and a glioma diagnosis method using the glioma recognizing antibody according to any of "23" to "25" ("30").

### Brief Description of Drawings

Fig. 1 is a photograph showing expression analysis results for the glioma antigen, KU-GB-3, by RT-PCR.
Fig. 2 is a photograph showing expression analysis results for the glioma antigen, KU-GB-4, by RT-PCR.
Fig. 3 is a photograph showing expression analysis results for SOX5 (sex determining region Y-BOX5) by RT-PCR.
Fig. 4 is a photograph showing expression analysis results for SOX6 (sex determining region Y-BOX6) by RT-PCR.
Fig. 5 is a photograph showing expression analysis results for zinc finger homeobox 1B by RT-PCR.
Fig. 6 is a photograph showing expression analysis results for M-phase phosphoprotein 1 by RT-PCR.
Fig. 7 is a photograph showing Western blot analysis results for the reaction of an IgG antibody in a glioma patient serum with a recombinant SOX6 HMG box protein.
Fig. 8 is a view showing ELISA results for the reaction of an IgG antibody in a glioma patient serum with a recombinant SOX6 HMG box protein. In the figure, the bar indicates the median and "P < 0.01" indicates the unpaired t test.
Fig. 9 is a view showing expression analysis results for SOX6 (sex determining region Y-BOX6) by RT-PCR.
Fig. 10 is a photograph showing Northern blot analysis results for SOX6 in a glioma tissue. The left panel shows the expression of SOX6 in normal tissue (testis, fetal brain, and adult brain) and the right panel shows the expression of SOX6 in a glioma tissue sample.
Fig. 11 is a photograph showing Western blot analysis results for SOX6 in a glioma tissue. The right panel shows the results after absorption reaction with the SOX6 antibody.
Fig. 12 is a photograph showing immunohistochemical analysis results for SOX6 expression in glioma and normal brain tissues.

### Best Mode of Carrying Out the Invention

As the present invention's testis-derived antigenic protein, having an immunity induction activity comparable to a glioma antigenic protein, an antigenic protein, obtained by a preparation method comprising the steps of: (1) extracting and purifying total RNA from testis tissue, synthesizing cDNA using the purified mRNA, and constructing a λ phage cDNA library by introducing the cDNA into a λ phage vector and infecting Escherichia coli with the vector; (2) diluting the serum of a glioma patient, reacting it with an extract of the Escherichia coli, and removing the reaction products to prepare a reaction serum; (3) reacting the abovementioned λ phage cDNA library with the reaction serum and using a labeled anti-IgG antibody to detect positive clones that react with the antibody in the serum; (4) repeating the screening process several times on the detected positive clones to confirm the antibody reactivity; and (5) isolating an antigen from the positive clones and performing serum screening using the isolated antigen and at least a glioma patient serum and a serum of a healthy individual; can be cited. Also, it is preferable to use PCR to amplify and determine the sequence of the cDNA insert in the positive clones obtained in the abovementioned step (4) and carry out a homology search using an existing gene database, etc., and in step (5), it is preferable to perform serum screening using, in addition to the glioma patient serum and the healthy individual serum, the sera of other brain disorder patients and other cancer patients.

As specific examples of this testis-derived antigenic protein, one or two or more testis-derived antigenic proteins that are selected from a group of glioma-specific antigens (glioma-specific antigenic genes), which react only with or exhibit a high reactivity to glioma patient sera and include KU-GB-3, KU-GB-4, SOX family molecules, such as SOX5 (sex determining region Y BOX6) (GenBank Accession No. NM_006940), SOX6 (GenBank Accession No. NM_033326), SOX13 (GenBank Accession No. NM_005686), etc., zinc finger homeobox (zinc finger homeobox 1B) (GenBank Accession No. NM_014795), an ADP-ribosylation factor (ADP-ribosylation factor 4-like) (GenBank Accession No. NM_033326), etc., and a group of glioma-nonspecific antigenes (glioma-nonspecific antigenic genes), which do not exhibit specificity in reactions with glioma patient sera and include M-phase phosphoprotein 1 (GenBank Accession No. NM_016195), etc., can be cited. Using this testis-derived antigenic protein, dendritic cells, which are powerful antigen-presenting cells, can be expressed in vivo or in vitro and immunity can be induced by the administration of these antigen-expressed dendritic cells.

Of the abovementioned testis-derived antigenic proteins having an immunity induction activity comparable to a glioma antigenic protein, new antigenic proteins and isoforms thereof can be cited as examples of the present invention's protein, with specific examples including the testis-derived antigenic protein KU-GB-3, indicated by SEQ ID NO. 2 in the sequence listing, the testis-derived antigenic protein KU-GB-4, indicated by SEQ ID NO. 4, and proteins, each comprising an amino acid sequence in which one or several amino acids have been deleted, substituted, or added with respect to the amino acid sequence indicated by SEQ ID NO. 2 or 4 and having an immunity induction activity. Here, immunity induction activity refers to the activity of inducing antibody production, cellular immunity, immunologic tolerance, and other immunological reactions, and with regard to such immunity induction activity, a protein that has a T cell induction activity and increases the incidence of precursor cells of cytotoxic T lymphocytes (CTLs) in peripheral blood is especially preferable.

As examples of the present invention's peptide, peptides, comprising a part of any of the abovementioned proteins, having an immunity induction activity, can be cited, and though this peptide is not restricted in particular, a peptide that makes up the recognizing part of an antibody or the recognizing part of a CD4⁺T cell and/or CD8⁺T cell can be cited as a preferable example. In addition to the abovementioned proteins and peptides that are subjects of the present invention, recombinant proteins and peptides that bind specifically with an antibody that binds specifically with an abovementioned protein or peptide are also included in the present invention, and these may be referred to collectively hereinafter as "the presently concerned glioma antigens." The origin of the presently concerned glioma antigens is not restricted to humans, and the presently concerned glioma antigens are useful as tumor detection diagnostic drugs and treatment drugs as well as for methods of screening immunity induction activity promoters or inhibitors, as shall be described below.

The DNAs that are subjects of the present invention include DNAs that encode the abovementioned proteins of the present invention, with examples including a DNA encoding the testis-derived antigen protein KU-GB-3, having the amino acid sequence indicated by SEQ ID NO. 2, a DNA encoding the testis-derived antigen protein KU-GB-4, having the amino acid sequence indicated by SEQ ID NO. 4, a DNA encoding a protein, comprising an amino acid sequence in which one or several amino acids have been deleted, substituted, or added with respect to the amino acid sequence indicated by SEQ ID NO. 2 or 4 and having an immunity induction activity, and a DNA, comprising the base sequence indicated by SEQ ID NO. 1 or 3 or a base sequence complementary to the same or a base sequence containing a part or the whole of the same. Oligonucleotide sequences, which can be used for amplification, and DNAs and RNAs, which contain a nucleotide sequence that can be hybridized under conditions enabling use as a probe or a marker, are also included in the present invention. These DNAs or RNAs can be used to examine variations of the expression amounts of the presently concerned glioma antigens for the diagnosis of glioma and other cancer disorders related to the expression or function of the presently concerned glioma antigens or the diagnosis of susceptibility to such a disorder.

Also, by using the base sequence indicated by SEQ ID NO. 1 or 3 or a base sequence complementary to the same or a base sequence containing a part or the whole of the same as a probe, performing hybridization under stringent conditions with a DNA library originating from various glioma cells, and isolating the DNA that hybridizes with the abovementioned probe, a DNA, which encodes a protein having an immunity induction activity and intended to be comparable in effect to such antigenic proteins as KU-GB-3 and KU-GB-4, can be obtained. The DNA that is thus obtained also falls within the scope of the present invention. As hybridization conditions for obtaining the present invention's DNA, hybridization at 42°C and washing treatment at 42°C by a washing buffer containing 1 × SSC and 0.1% SDS can be cited, and hybridization at 65°C and washing treatment at 65°C by a washing buffer containing 0.1 × SSC and 0.1% SDS can be cited as more preferable conditions. The factors that affect the stringency of hybridization include various factors besides the abovementioned temperature conditions, and those skilled in the art can realize stringency of a level equivalent to the stringency of hybridization conditions given above as examples by appropriate combination of the various factors.

The present invention's oligonucleotide or peptide nucleic acid is not restricted in particular as long as it hybridizes specifically with a DNA having the base sequence indicated by SEQ ID NO. 1 or 3 or a base sequence complementary to the same or a base sequence containing a part or the whole of the same or an RNA corresponding to an abovementioned DNA and can inhibit the expression of a presently concerned glioma antigen (see for example, Bioconjugate Chem. Vol. 5, No. 1, 1994). The abovementioned "peptide nucleic acid" refers to a chemically synthesized nucleic acid analog, which takes the place of a nucleic acid (DNA/RNA), constructed by substituting the pentose/phosphate backbone that is the basic backbone structure of nucleic acid with a polyamide backbone with glycine as a unit, and has a three-dimensional structure similar to a nucleic acid. Such a peptide nucleic acid is preferable in terms of inhibiting the expression of a presently concerned glioma antigen since its bases bind extremely specifically and strongly with a nucleic acid having a complementary base sequence. The abovementioned "oligonucleotide" refers to an oligonucleotide, formed from naturally occurring bases or sugar parts that are bound by a proper phosphodiester bond, etc., or an analog of the same. An oligonucleotide, formed from naturally occurring bases or sugar parts that are bound by a proper phosphodiester bond, etc., refers to a naturally occurring species, a naturally occurring subunit, or a synthetic species formed from a homolog of such a naturally occurring species or naturally occurring subunit. The abovementioned subunit refers to a base-sugar combination bound to an adjacent subunit by a phosphodiester bond or other type of bond.

An analog of an oligonucleotide formed from naturally occurring bases or sugar parts that are bound by a proper phosphodiester bond, etc., refers to an oligonucleotide that functions in a comparable manner to the abovementioned oligonucleotide but has a base that does not occur naturally, for example, a modified base, such as dihydrouridine, inosine, 4-acetylcytidine, 1-methyladenosine, etc., or is increased in stability by chemical modification of the phosphate groups, sugar parts, or the 3' and 5' terminals, etc. Examples of methods of chemical modification of the phosphate groups, sugar parts, or the 3' and 5' terminals, etc., include but are not limited to the method of substituting one of the oxygen atoms of the phosphodiester group between nucleotides by sulfur and thereby obtaining an oligophosphorothioate, the method of substituting one of the oxygen atoms of the phosphodiester group between nucleotides by -CH₃ and thereby obtaining an oligomethylphosphonate, the method of substituting a phosphodiester bond part by another structure that is nonionic and nonchiral, etc.

The abovementioned oligonucleotide may be produced by one skilled in the art by a known synthesis method, for example, by a solid-phase synthesis method using a synthesis device made by Applied Biosystems Inc., etc. Also, other oligonucleotide analogs, such as phosphorothioates and alkylated derivatives may be produced using similar methods (Akira Murakami et al., "Chemical synthesis of functional antisense DNA," Organic Synthetic Chemistry, 48(3): 180-193, 1990). The present invention's oligonucleotides and peptide nucleic acids that are obtained by such methods can restrain the forming of the presently concerned glioma antigens in animals.

Also, as shall be described in detail below, the abovementioned gene or DNA encoding a presently concerned glioma antigen or a presently concerned glioma antigen, etc., can be used to prepare a fusion peptide or fusion protein, wherein a presently concerned glioma antigen is bound with a marker protein and/or a peptide tag, an antibody against a presently concerned glioma antigen, a recombinant vector containing a DNA encoding a presently concerned glioma antigen, a host cell, containing an expression system that can express a presently concerned glioma antigen, a non-human animal, in which a genetic function encoding a presently concerned glioma antigen is absent from the chromosomes, or a non-human animal, which overexpresses a presently concerned glioma antigen.

The present invention's fusion protein or fusion peptide may be any fusion peptide or fusion protein as long as a presently concerned glioma antigen is bound with a marker protein and/or a peptide tag. Specific examples of marker proteins include but are not limited to priorly known marker proteins, such as alkaline phosphatase, the Fc region of an antibody, HRP, GFP, etc., and specific examples of peptide tags include but are not limited to priorly known peptide tags such as His tags, FLAG tags, S tags, etc. Such a fusion protein can be prepared by a routine procedure and is useful for the purification of an antigen protein, such as KU-GB-3, KU-GB-4, etc., by use of the affinity of Ni-NTA and a His tag, the detection of proteins having T cell induction activity, the quantification of an antibody against an antigen protein, such as KU-GB-3, KU-GB-4, etc., and is also useful as a diagnostic marker for glioma or other type of cancer or a research reagent in the field of the art.

Immune specific antibodies, such as monoclonal antibodies, polyclonal antibodies, chimeric antibodies, straight-chain antibodies, humanized antibodies, etc., can be cited as specific examples of the present invention's antibody against a presently concerned glioma antigen, and though these can be prepared by routine procedures using the presently concerned glioma antigens mentioned above, monoclonal antibodies are preferable among such antibodies in terms of specificity, and in particular, a monoclonal antibody that specifically recognizes the epitope of KU-GB-3 or KU-GB-4, etc., or a complex of such an epitope and HLA is more preferable. Such monoclonal antibodies and other antibodies are useful not only for the diagnosis of glioma and other cancers and missile therapy and other treatment methods but are also useful for clarifying the initiation mechanisms of glioma and other malignant tumors.

The present invention's antibody is produced using conventional protocols and by administering, to an animal (preferably besides a human), a presently concerned glioma antigen or a fragment thereof that contains an epitope, or cells, on the membrane surface of which is expressed a presently concerned glioma antigen, in particular, a complex of an epitope and HLA. For example for the preparation of a monoclonal antibody, an optional method that provides an antibody produced by a continuous cell culture, such as the hybridoma method (Nature 256, 495-497, 1975), trioma method, human B cell hybridoma method (Immunology Today 4, 72, 1983), and EBV-hybridoma method (MONOCLONAL ANTIBODIES AND CANCER THERAPY, pp.77-96, Alan R. Liss, Inc., 1985), may be used.

For preparation of the abovementioned straight-chain antibody against a presently concerned glioma antigen, a method of preparing a straight-chain antibody (United States Patent No. 4,946,778) may be applied. A transgenic mouse or other mammal can be used to express a humanized antibody, and using this antibody, a clone that expresses the presently concerned glioma antigen can be isolated and identified or the polypeptide can be purified using affinity chromatography. There is a possibility for an antibody against the presently concerned glioma antigens and peptides containing epitopes thereof to be used for the diagnosis and treatment of glioma, etc. Recombinant proteins or peptides to which such antibodies bind specifically are also included as mentioned above among the presently concerned glioma antigens of the present invention.

The present invention's recombinant vector is not restricted in particular as long as it is a vector that contains a DNA encoding a presently concerned glioma antigen, and a vector, containing an expression system that can make a presently concerned glioma antigen be expressed inside a host cell, is preferable. Examples of such a vector include vectors containing an expression system derived from a chromosome, episome, or virus, and to be more specific, include bacterial plasmid derived vectors, yeast plasmid derived vectors, vectors derived from SV40 and other papova viruses, vaccinia viruses, adenoviruses, chicken pox viruses, pseudorabies viruses, and retroviruses, and vectors derived from bacteriophages, transposons, and combinations of these, such as a vector derived from the genetic elements of a cosmid, phagemid, or other plasmid and a bacteriophage. The expression system may contain not only a sequence that causes expression but may also contain a control sequence that regulates the expression.

Host cells, containing an expression system that can express a presently concerned glioma antigen of the present invention, may be prepared by introducing a gene encoding the presently concerned glioma antigen in host cells, and such introduction of a DNA encoding a presently concerned glioma antigen, a vector containing such a DNA, or other expression system mentioned above into host cells can be carried out by methods described in various standard laboratory manuals, such as that by Davis et al. (BASIC METHODS IN MOLECULAR BIOLOGY, 1986) and Sambrook et al. (MOLECULAR CLONING: A LABORATORY MANUAL, 2nd Ed., Cold Spring Harbor Laboratory Press, Cold Spring Harbor, N.Y., 1989), that is for example, calcium phosphate transfection, DEAE-dextran mediated transfection, transvection, microinjection, cationic resin mediated transfection, electroporation, transduction, scrape loading, ballistic introduction, infection etc. Though as host cells, prokaryotic cells of bacteria, such as Escherichia coli, streptomyces, Bacillus subtilis, streptococcus, staphylococcus, etc., cells of fungi, such as yeast, aspergillus, etc., insect cells, such as Drosophila S2, Spodoptera Sf9, animal cells, such as L cells, CHO cells, COS cells, HeLa cells, C127 cells, BALB/c3T3 cells (including mutant cell lines lacking dihydrofolic acid reductase, thymidine kinase, etc.), BHK21 cells, HEK293 cells, Bowes malignant melanoma cells, etc., plant cells, etc., can be cited, human cells are preferable.

Also, prokaryotic cells of bacteria, such as Escherichia coli, streptomyces, Bacillus subtilis, streptococcus, staphylococcus, etc., cells of fungi, such as yeast, aspergillus, etc., insect cells, such as Drosophila S2, Spodoptera Sf9, animal cells, such as L cells, CHO cells, COS cells, HeLa cells, C127 cells, BALB/c3T3 cells (including mutant cell lines lacking dihydrofolic acid reductase, thymidine kinase, etc.), BHK21 cells, HEK293 cells, etc., plant cells, etc., can be cited as examples of the abovementioned host cells. Also, host cells, having HLA expression ability and containing an expression system that can express a presently concerned glioma antigen, can be prepared by introducing a gene encoding the presently concerned glioma antigen by an abovementioned method into host cells having an HLA expression ability or host cells, which do not originally have an HLA expression ability but into which HLAcDNA has been transfected.

The expression system may be any expression system that can make a presently concerned glioma antigen be expressed inside host cells, and may be an expression system derived from chromosomes, episomes, and viruses, such as bacterial plasmid derived vectors, yeast plasmid derived vectors, vectors derived from SV40 and other papova viruses, vaccinia viruses, adenoviruses, adeno-associated viruses, chicken pox viruses, pseudorabies viruses, and retroviruses, and vectors derived from bacteriophages, transposons, and combinations of these, such as a vector derived from the genetic elements of a cosmid, phagemid, or other plasmid and a bacteriophage. This expression system may contain not only a sequence that causes expression but may also contain a control sequence that regulates the expression.

Host cells containing an abovementioned expression system or the cell membranes of such cells or the presently concerned glioma antigens that are obtained by culturing such cells can be used in the present invention's screening method as shall be described later. As a method of obtaining cell membranes, the method of F. Pietri Rouxel et al. (Eur. J. Biochem., 247, 1174-1179, 1997), etc., may be used, and for the recovery and purification of such a presently concerned glioma antigen from a cell culture, known methods, including ammonium sulfate or ethanol precipitation, acidic extraction, anionic or cationic exchange chromatography, phosphocellulose chromatography, hydrophobic interaction chromatography, affinity chromatography, hydroxyapatite chromatography, and lectin chromatography, may be used, and high-performance liquid chromatography is preferably used. Particularly in regard to a column to be used in affinity chromatography, a presently concerned glioma antigen can be obtained with a column to which an antibody against the presently concerned glioma antigen is bound, or, in an abovementioned case where a commonly-used peptide tag is added to the presently concerned glioma antigen, with a column to which a substance that has affinity to the peptide tag is bound.

The present invention's non-human animal, in which a genetic function encoding a presently concerned glioma antigen is absent from the chromosomes, refers to a non-human animal, which has lost the function of expressing the presently concerned glioma antigen by inactivation by destruction, loss, substitution or other genetic mutation of a part or the whole of a gene encoding the presently concerned glioma antigen on the chromosomes. The present invention's non-human animal, which overexpresses a presently concerned glioma antigen refers to a non-human animal that produces the presently concerned glioma antigen in large amounts in comparison to a wild type non-human animal. Examples of the abovementioned non-human animal include but are not limited to rodents, such as mice, rats, etc., and other non-human animals.

Homozygous non-human animals that are born according to Mendel's law include both a type lacking or overexpressing a presently concerned glioma antigen and a wild type littermate, and precise comparative experiments at an individual level can be conducted by simultaneously using the type lacking or overexpressing the presently concerned glioma antigen and wild type littermate. It is thus preferable to use, along with the non-human animal, in which the genetic function encoding a presently concerned glioma antigen is absent from the chromosomes or is overexpressed, a wild type non-human animal, that is an animal of the same species or an animal of the same brood in carrying out, for example, the present invention's screening to be described below. Methods of preparing a non-human animal, in which a genetic function encoding a presently concerned glioma antigen is absent from the chromosomes or is overexpressed, shall now be described using a knockout mouse or transgenic mouse for a presently concerned glioma antigen as an example.

For example, to prepare mice, in which a genetic function encoding a presently concerned glioma antigen is absent from the chromosomes, that is, knockout mice for a presently concerned glioma antigen, a gene encoding the presently concerned glioma antigen is screened using gene fragments obtained by PCR or other method from a mouse gene library, and the screened gene encoding the presently concerned glioma antigen is subcloned using a viral vector, etc., and specified by DNA sequencing. The whole or part of this clone's gene encoding the presently concerned glioma antigen is then substituted in a pMC1 neo gene cassette, etc., and a targeting vector is prepared by introducing a gene, such as a diphtheria toxin A fragment (DT-A) gene or a herpes simplex virus thymidine kinase (HSV-tk) gene, into the 3'-terminal side.

The prepared targeting vector is linearized and then introduced into ES cells by a method such as electroporation. The ES cells are then homologously recombined, and from among the homologous recombinants, ES cells, in which homologous recombination was caused by G418, ganciclovir (GANC) or other such antibiotics, are selected. It is preferable to confirm by Southern blotting etc., whether these selected ES cells are the desired recombinants. Clones of the confirmed ES cells are then microinjected into mouse blastocysts, and the blastocysts are transplanted into a recipient mouse to generate chimeric mice. Heterozygous mice can be obtained by intercrossing the chimeric mice with wild-type mice, and knockout mice for the presently concerned glioma antigen can be obtained by intercrossing the heterozygous mice. In addition, as a method for confirming whether knockout mice for the presently concerned glioma antigen have been obtained, for example, RNA is isolated from mice obtained by the above-described method and examined by Northern blotting, etc., or the expression of the presently concerned glioma antigen in the mice is examined by Western blotting, etc.

Also transgenic mice for the present invention's glioma antigen can be prepared by constructing a transgene by fusing cDNA, encoding a presently concerned glioma antigen, with a chicken β-actin, mouse neurofilament, or SV40 promoter etc., and a rabbit β-globin or SV40 polyA or intron etc., microinjecting the transgene into the pronucleus of mouse fertilized egg, culturing and then transplanting the obtained egg cells into the oviduct of a recipient mouse, and thereafter tending the recipient animal and selecting infant mice having the abovementioned cDNA from among the infant mice that are born. The selection of infant mice with the cDNA can be carried out by extracting crude DNA from the tails, etc., of the mice and performing dot hybridization using the introduced gene, encoding the presently concerned glioma antigen, as a probe or performing PCR using a specific primer, etc.

The present invention's method for screening an immunity induction activity promoter or inhibitor is not restricted in particular as long as it uses a presently concerned glioma antigen, a test substance, and T cells to measure and evaluate immunity induction activity in T cells, and a method of using a test substance, a presently concerned glioma antigen, and T cells to measure and evaluate immunity induction activity in the abovementioned T cells, a method of using a test substance, cell membranes or cells expressing a presently concerned glioma antigen, and T cells to measure and evaluate immunity induction activity in the abovementioned T cells, a method of using host cells, which express a presently concerned glioma antigen and have been transfected by both an HLA-expressing vector and a vector expressing a peptide to be tested, and T cells to measure and evaluate immunity induction activity in the abovementioned T cells, a method of using host cells, which express a presently concerned glioma antigen, have an HLA expressing ability, and have been transfected by a vector expressing a polypeptide to be tested, and T cells to measure and evaluate immunity induction activity in the abovementioned T cells, a method of administering a test substance to the above-described knockout mouse, transgenic mouse, or other non-human animal to measure and evaluate the immunity induction activity of the T cells in the non-human animal, etc., can be cited as examples. Specific examples of the abovementioned cell membranes or cells include cells, such as primary cultured cells obtained from a non-human animal, which overexpress the abovementioned gene encoding a presently concerned glioma antigen, or a wild type non-human animal, etc., host cells, containing the abovementioned expression system that can express a presently concerned glioma antigen, and cell membranes of such cells, etc. As a method of putting such cell membranes or cells into contact with a test substance, a method of culturing cell membranes or cells expressing a presently concerned glioma antigen in vitro, in the presence of the test substance, and then putting them into contact with T cells, etc., can be cited as examples. As a method of measuring and evaluating immunity induction activity in T cells, a method of evaluating the amount of IFNγ, which is released from the T cells to a medium, as an index can be cited as a specific example. An immunity induction activity promoter obtained by the above-described screening method can be used for treatment of a patient requiring the promotion of immunity induction activity, etc., and an immunity induction activity inhibitor obtained by the above-described screening method can be used for treatment of a patient requiring the inhibition of immunity induction activity, etc.

The present invention's glioma detection diagnostic drug is not restricted in particular as long as it is a reagent containing a testis-derived antigen protein or part thereof that has an immunity induction activity comparable to the present invention's glioma antigen protein. An antigen recognition part of a glioma antigen, etc., can be cited as an example of a part of an antigen protein, and in a case where two or more types of antigens are to be used in combination, the antigens are preferably selected from among KU-GB-3, KU-GB-4, SOX family molecules, such as SOX5 (sex determining region Y BOX6) (GenBank Accession No. NM_006940), SOX6 (GenBank Accession No. NM_033326), SOX13 (GenBank Accession No. NM_005686), etc., zinc finger homeobox (GenBank Accession No. NM_014795), ADP-ribosylation factors (GenBank Accession No. NM_033326) and other proteins of the glioma-specific antigen group or M-phase phosphoprotein 1 and other proteins of the glioma-nonspecific antigen group. Reagents, containing a monoclonal antibody or other antibody that specifically binds to the abovementioned testis-derived antigen protein or part thereof that has an immunity induction activity comparable to the present invention' s glioma antigen protein, may also be used as the present invention's glioma detection diagnostic drug, and even with these antibodies, in a case where two or more such antibodies are to be used in combination, selection is preferably made from among antibodies against KU-GB-3, KU-GB-4, SOX family molecules, such as SOX5 (sex determining region Y BOX6), SOX6, SOX13, etc., zinc finger homeobox, ADP-ribosylation factors and other proteins of the glioma-specific antigen group or M-phase phosphoprotein 1 and other proteins of the glioma-nonspecific antigen group. Examples of methods of diagnosing glioma or other cancers using the present invention's glioma detection diagnostic drug include a method of using a labeled glioma antigen and making it react with an IgG antibody in a serum obtained from a test subject to examine whether or not an immune reaction is seen, a method of administering an antibody, which is fluorescence labeled or labeled with iron, etc., and is an antibody against the abovementioned testis-derived antigen protein or part thereof that has an immunity induction activity comparable to the present invention's glioma antigen protein, into blood and carrying out image diagnosis of glioma or other cancer with unclear boundaries, etc.

The present invention's glioma detection/diagnosis probe is not restricted in particular as long as it is a reagent containing an antisense chain of a DNA or RNA encoding the abovementioned testis-derived antigen protein or part thereof that has an immunity induction activity comparable to the present invention's glioma antigen protein. Specific examples include reagents containing a part or the whole of an antisense chain of a DNA or RNA encoding one or two or more types of glioma antigen selected from among KU-GB-3, KU-GB-4, SOX family molecules, such as SOX5 (sex determining region Y BOX6), SOX6, SOX13, etc., zinc finger homeobox, ADP-ribosylation factors and other proteins of the glioma-specific antigen group or M-phase phosphoprotein 1 and other proteins of the glioma-nonspecific antigen group, and a DNA or RNA of 20 bp or more is preferable, and in a case where two or more probes are to be used in combination, the probes are preferably selected from among the abovementioned antisense chains of DNA or RNA encoding one or two or more types of antigen proteins selected from among KU-GB-3, KU-GB-4, SOX family molecules, such as SOX5 (sex determining region Y BOX6), SOX6, SOX13, etc., zinc finger homeobox, ADP-ribosylation factors and other proteins of the glioma-specific antigen group or M-phase phosphoprotein 1 and other proteins of the glioma-nonspecific antigen group. As an example of a glioma detection/diagnosis method using the present invention's glioma detection/diagnosis probe, a method of using a labeled antisense chain to detect the mRNA of the abovementioned testis-derived antigen protein or part thereof that has an immunity induction activity comparable to the present invention's glioma antigen protein obtained from a test subject, can be cited. Specific examples of the test subject used in this detection/diagnosis method include but are not restricted to genome DNA, RNA, and cDNA that can be obtained from cells of a subject, for example, cells in blood, urine, saliva, tissue, and other samples obtained by biopsy.

As the present invention's antitumor agent, a preparation containing, as an effective component thereof, the whole or a part of one type or two or more types of antigen protein obtained by the abovementioned method of preparing the present invention's testis-derived antigen protein having an immunity induction activity comparable to a glioma antigen protein, can be used. An antigen recognizing part, etc., of a glioma antigen can be cited as an example of a part of an antigen protein, and in a case where two or more antigens are to be used in combination, the antigens are preferably selected from among KU-GB-3, KU-GB-4, SOX family molecules, such as SOX5 (sex determining region Y BOX6), SOX6, SOX13, etc., zinc finger homeobox, ADP-ribosylation factors and other proteins of the glioma-specific antigen group or M-phase phosphoprotein 1 and other proteins of the glioma-nonspecific antigen group. Also as the present invention's antitumor agent, a preparation, containing, as an effective component thereof, an antibody, such as a monoclonal antibody, polyclonal antibody, chimeric antibody, straight-chain antibody, humanized antibody, etc., that binds specifically with the whole or a part of the abovementioned glioma antigen, can be used, and even with such antibodies, when two or more such antibodies are to be used in combination, selection is preferably made from among antibodies against KU-GB-3, KU-GB-4, SOX family molecules, such as SOX5 (sex determining region Y BOX6), SOX6, SOX13, etc., zinc finger homeobox, ADP-ribosylation factors and other proteins of the glioma-specific antigen group or M-phase phosphoprotein 1 and other proteins of the glioma-nonspecific antigen group. In using the present invention's antitumor agent, various commonly-used formulation components for drug preparation that are pharmaceutically allowed, such as carriers, binders, stabilizers, fillers, diluents, pH buffers, disintegrators, dissolution agents, solubilizing agents, isotonic agents, etc., may be added. These treatment drugs and biophylactic enhancement drugs may be administered orally or parenterally. That is, the drugs may be administered by a commonly employed form of administration, for example, parenteral administration by injection of a preparation in the form of a solution, emulsion, suspension, etc., or oral administration of a preparation in the form of a powder, granules, capsule, syrup, suspension, etc. In the case of oral administration, the antitumor agent is preferably prepared in an abovementioned liposome sealed/embedded form. The present invention's treatment method for glioma or other cancer disorder is not restricted in particular as long as it is a treatment method that uses the abovementioned antitumor agent, and antitumor effects due to increases in vivo activation of T cell induction can be anticipated by the administration of this antitumor agent orally or by intravenous, intracutaneous, or subcutaneous injection, etc. Also, T cells can be stimulated in vitro and induced to become activated T cells using the present invention's antitumor agent, and for example, when peripheral blood lymphocytes or tumor-infiltrated lymphocytes are stimulated by IL-2 and the present invention's antitumor agent, tumor reactive activated T cells are induced and these activated T cells can be used effectively in adoptive immunotherapy. The present invention's monoclonal antibodies and other antibodies can also be used to perform a genetic treatment, wherein a gene is selectively introduced into glioma, a missile therapy, wherein an antitumor agent is made to act selectively on glioma, etc.

Though the present invention shall now be described more specifically by way of examples, the technical scope of the present invention is not limited to these examples.

### Example 1 (Identification of testis-derived human glioma antigen by SEREX)

### [Cell lines and tissues]

Human glioma cell lines (GI-1, T98G, U87MG, U251, A172), a human malignant melanoma cell line (888MEL), lung cancer cell lines (LU99, EBC1), an esophageal cancer cell line (TE10), a pancreatic cancer cell line (PK1), a bladder cancer cell line (KU7), a prostatic cancer cell line (PC3), a breast cancer cell line (MDA231), a leukemia cell line (MOLT4), and fibroblasts were respectively cultured in RPMI, containing 10% bovine fetus serum along with penicillin (100 IU/ml) and streptomycin (100 µg/ml). As human glioma tissues [GB (glioblast) 13, GB17, GB16, GB4], parts of lesions extracted by surgery were used. RNAs of normal tissues (brain, heart, lung, stomach, small intestine, large intestine, liver, spleen, kidney, testis, placenta, muscle, fetal brain) were purchased from Clontech Laboratories Inc.

### [Construction of cDNA library]

Total RNA of adult human testis was purchased from Clontech Laboratories Inc. Poly(A)⁺RNA was purified twice using latex beads coated with oligodeoxythymidylic acid (Oligotex-dT30 super; Takara Shuzo Co., Ltd.). By reverse transcription using 5µg of the poly(A)⁺RNA and the ZAP-cDNA Synthesis Kit (Stratagene Inc.), a testis cDNA library was constructed. Upon insertion of cDNA fragments into the bacteriophage expression vector, λzapII (Stratagene Inc.), 3 × 10⁶ primary recombinants were obtained as the library.

### [Screening of testis cDNA library by serum]

The cDNA library constructed as described above was sown at an amount of 1 × 10⁴ clones in a 150 mm NZY agarose plate and incubated at 42°C for 4 hours, followed by further incubation at 37°C for 4 hours. Recombinant proteins, expressed and induced by 10 mM of IPTG on Escherichia coli (XL1-Blue), were transferred onto nitrocellulose filters (Hybond-c; Amersham, Buckinghamshire, England), and after removing the adsorbed bacteria and phages by washing the filters with TBS (10 mM of Tris-NCl, 150 mM of NaCl; pH 7.5), containing 0.05% of TWIN 20, nonspecific reactions were inhibited with TBS containing 5% skim milk. The filters were then reacted at 37°C for 4 hours with patient sera diluted by 1:400. As patient sera, sera were sampled respectively from the twelve glioma patients shown in Table 1 and mixtures, each of sera of four glioma patients [(mixture of patient sera G1, G3, G5, and G8), (mixture of patient sera G20, G21, G22, and G27), (mixture of patient sera G18, G23, G24, and G28)], were used. These patient sera were stored at -80°C, and immediately prior to use, each serum was diluted by 1:5 with a TBS solution containing 5% skim milk. In order to priorly eliminate antibodies that react with bacteria, each serum was mixed at a ratio of 1:2 with a lysate of lysed Escherichia coli and reacted at 4°C for 8 hours, the resulting supernatant was diluted by 1:400 in the final stage, and this diluted supernatant was used as the serum.

**(Table 1)**

| Patient serum | Age | Sex | Diagnosis | Number of types of isolated gene |
|---|---|---|---|---|
| G1 | 48 | M | Glioblastoma | |
| G3 | 53 | F | Glioblastoma | 3 (including 2 new |
| G5 | 61 | M | Anaplastic oligoastrocytoma | types) |
| G8 | 30 | M | Anaplastic astrocytoma | |
| | | | | |
| G20 | 17 | F | Anaplastic astrocytoma | |
| G21 | 32 | M | Astrocytoma | 8 (including 2 new |
| G22 | 18 | M | Anaplastic astrocytoma | types) |
| G27 | 25 | M | Astrocytoma | |
| | | | | |
| G18 | 21 | F | Glioblastoma | |
| G23 | 3 | F | Astrocytoma | 4 |
| G24 | 7 | F | Oligoastrocytoma | |
| G28 | 41 | M | Anaplastic oligoastrocytoma | |
| | | | | Total 15 |

The sera that were thus processed were then reacted at 37°C for 4 hours with the recombinant proteins that were plotted on the nitrocellulose filters, and the recombinant proteins that reacted with the antibodies in sera were incubated with alkaline phosphatase-labeled goat antihuman IgG (Fc) antibody (Cappel Inc.). Enzymatic detection of bound secondary antibodies was then carried out using nitro blue tetrazolium (Boehringer Mannheim GmbH) and 5-bromo-4-chloro-3-indolyl phosphate (Sigma Chemical Co.). Positive clones corresponding to the parts of positive color reaction were then sampled from the abovementioned 150 mm NZY agarose plate and dissolved in SM buffer solution (100 mM of NaCl, 10 mM of MgSO₄, 50m M of Tris-HCl, 0.01% of gelatin; pH 7.5). This screening process was repeated several times to confirm the antibody reactivity of the positive clones. 58 positive clones were isolated by screening from 5 × 10⁵ phage clones per one patient's serum. [Homology search of isolated antigen gene]

The cDNA inserts that were incorporated in the 58 phages obtained as described above were then amplified by PCR using the ExTaq kit (Takara Shuzo Co., Ltd.) and the respective base sequences were determined. ExTaq (Takara Shuzo Co., Ltd.) was used as the reaction enzyme, T3 (5'-AATTAACCCTCACTAAAGGG-3' ; SEQ ID NO. 5) was used as the sense primer, and T7 (5'-GTAATACGACTCACTATAGGGC-3'; SEQ ID NO. 6) was used as the antisense primer. In regard to reaction conditions, a thermal cycler (Perkin Elmer) was used to perform denaturing at 94°C for 5 minutes just at first and then to repeat a cycle of heat denaturing at 94°C for 1 minute, annealing at 55°C for 1 minute, and extension at 72°C for 2 minutes 35 times. Lastly, extension at 72°C was carried out for 7 minutes. The PCR products obtained were subject to DNA sequencing using the Big Dye DNA Sequencing Kit (ABI) and the ABI310 Auto Sequencer and the base sequences at the 5' side were determined across a length of approximately 300 to 500 bp. The DNA thus determined were compared with the genetic information registered in the genetic databases of the National Center for Biotechnology Information or the EST databases to search for homologies with known genes. As a result, it was found that the abovementioned 58 positive clones were of fifteen types of antigen cDNA consisting of eleven types of cDNAs encoding known proteins and four types of cDNA encoding new proteins. The numbers of types of the fifteen types of antigen cDNA are shown according to patient in Table 1.

### [Serum screening]

In order to examine application to serum diagnosis of glioma, screening was performed using the respective clones that express the isolated fifteen types of antigens and using 1:100 diluted sera of 29 glioma patients (12 glioblastoma patients, 7 anaplastic astrocytoma patients, 4 anaplastic oligoastrocytoma patients, 4 astrocytoma patients, 2 oligoastrocytoma patients), 14 other brain disorder patients (2 malignant lymphoma patients, 1 brain abscess patient, 1 subarachnoid hemorrhage patient, 2 Parkinson's disease patients, 4 meningioma patients, 1 neurocytoma patient, 1 craniopharyngioma patient, 2 metastatic brain tumor patients), and 37 healthy individuals and the sera were examined to see whether or not IgG antibodies against the respective isolated glioma antigens can be detected. As a result, six types of glioma specific antigens (glioma specific antigen genes), which react only with glioma patient sera or react with many of the glioma patient sera but hardly undergo an antibody reaction with the sera of healthy individuals, and one type of glioma nonspecific antigen (glioma nonspecific antigen gene), for which specificity was not found for reaction with glioma patient sera but which does react with glioma patient sera, were obtained. As shown in Table 2, the six types of glioma specific antigens (glioma specific antigen genes), including the new antigen, KU-GB-4, were KU-GB-3, SOX5 (sex determining region Y BOX6), SOX6, zinc finger homeobox, and an ADP ribosylation factor. Also, as shown in Table 3, the one type of glioma nonspecific antigen (glioma nonspecific antigen gene) was M-phase phosphoprotein 1. Though the abovementioned M-phase phosphoprotein 1 did not exhibit specificity in reactions with sera, the six types of glioma specific antigens were found to react specifically with glioma patient sera and are thus considered to be useful as a serum diagnostic drugs or treatment drugs for glioma.

Also as shown in Table 2, screening was performed using separately isolated clones expressing SOX13 and using 1:100 diluted sera of 29 glioma patients (12 glioblastoma patients, 7 anaplastic astrocytoma patients, 4 anaplastic oligoastrocytoma patients, 4 astrocytoma patients, 2 oligoastrocytoma patients) to examine whether or not IgG antibodies against the respective isolated glioma antigens can be detected in these sera. As a result, reactions were detected with exactly the same patient sera as those reacting with SOX5 and SOX6. As with SOX5 and SOX6, SOX13 is a molecule belonging to the SOX D group of the SOX family, and these SOX D group molecules are considered to be useful as serum diagnostic drugs or treatment drugs for glioma.

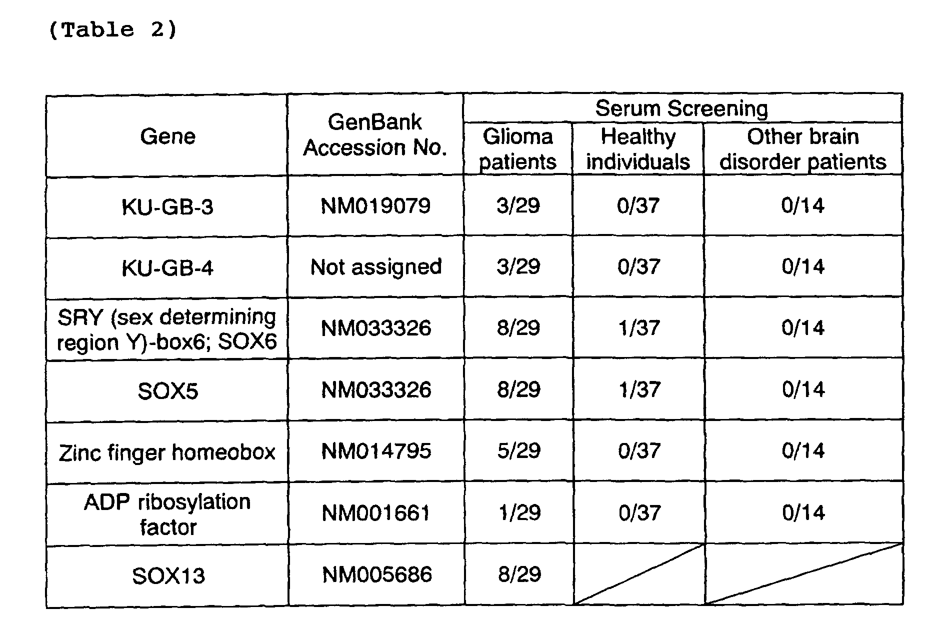

**(Table 3)**

| Gene | GenBank Accession No. | Serum Screening | | |
|---|---|---|---|---|
| | | Glioma patients | Healthy individuals | Other brain disorder patients |
| M-phase phosphoprotein 1 | NM016195 | 2/29 | 2/37 | 0/14 |

### [Determination of the base sequences and amino acid sequences of the antigens]

As a result of examining the base sequences of the isolated clones using an automatic DNA sequencer (ALF Express; Pharmacia Biotech Inc.), the KU-GB-3 gene was found to comprise a base sequence (SEQ ID NO. 1) with a total length of 2898 bp containing a gene encoding a sequence of 865 amino acids (SEQ ID NO. 2), and the KU-GB-4 gene was found to comprise a base sequence (SEQ ID NO. 3) with a total length of 1748bp containing a gene encoding a sequence of 399 amino acids (SEQ ID NO. 4). Though the KU-GB-3 gene had been registered as a placenta-derived cDNA in GenBank (Accession No. AK001973), it was not known as a glioma antigen.

### [RT-PCR method for detecting the expression of glioma antigens]

The expression specificities of the genes of the glioma antigens KU-GB-3, KU-GB-4, SOX5, SOX6, zinc finger homeobox, and M-phase phosphoprotein 1 in normal tissues, glioma cell lines and tissues, and other tumor cell lines were examined by the RT-PCR method. 5 µg each of RNAs derived from the normal tissues of brain, heart, lung, stomach, large intestine, liver, spleen, kidney, testis, placenta, muscle, fetal brain, etc., (all purchased from Clontech Laboratories Inc.), four types of glioma tissues (GB13, GB17, GB16, and GB4), and four types of glioma cell lines (GI-1, U87MG, T98G, U251, and A172) and an RNA derived from cultured fibroblasts were used along with avian myeloblast virus reverse transcriptase (Takara Co., Ltd.) and oligo (dT) as the primer to prepare a cDNA panel to serve as a mold for RT-PCR at a total reaction amount of 200 µl at 42°C. For the RT-PCR method for detection of expression of the KU-GB-4 gene, 5 µg each of RNAs derived from a malignant melanoma cell line (888MEL), lung cancer cell lines (LU99, EBC1), an esophageal cancer cell line (TE10), a pancreatic cancer cell line (PK1), a bladder cancer cell line (KU7), a prostatic cancer cell line (PC3), a breast cancer cell line (MDA231), and a leukemia cell line (MOLT4) were used.

5'-TCAAGGAACTGGCATACCCTGTCT-3' (SEQ ID NO. 7: P1) was used as the sense primer and 5'-GGTACTCGACAAGCACTGACAATC-3' (SEQ ID NO. 8: P2) was used as the antisense primer for detection of KU-GB-3, 5'-CAAACTCTGGGAGGAAGACC-3' (SEQ ID NO. 9: P3) was used as the sense primer and 5'-AGTTGGCTGAGAAGGACAAG-3' (SEQ ID NO. 10: P4) was used as the antisense primer for detection of KU-GB-4, 5'-GCAACAGGAGGAAGGAAAT-3' (SEQ ID NO. 11: P5) was used as the sense primer and 5'-GACAAGCCAACTGATAAGGGTC-3' (SEQ ID NO. 12: P6) was used as the antisense primer for detection of SOX5, 5'-CGCGCTTTGAGAATTTGGGGCC-3' (SEQ ID NO. 13: P7) was used as the sense primer and 5'-TCTTTGTTGGGGAGGGGGGTGA-3' (SEQ ID NO. 14: P8) was used as the antisense primer for detection of SOX6, 5'-TGGAGATCACTCCATGGACGATAG-3' (SEQ ID NO. 15: P9) was used as the sense primer and 5'-CCTGTTCTTTGAAGCACCCATGT-3' (SEQ ID NO. 16: P10) was used as the antisense primer for detection of zinc finger homeobox, and 5'-AACGAGCCAAACGGAA-3' (SEQ ID NO. 17: P11) was used as the sense primer and 5'-GTATCCATCCCCTCAAGC-3' (SEQ ID NO. 18: P12) was used as the antisense primer for detection of M-phase phosphoprotein 1. For the detection of β-actin (551 bp) as the control, 5'-GTCGACAACGGCTCCGGCATGTGCA-3' (SEQ ID NO. 19: P13) was used as the sense primer and 5'-GGATCTTCATGAGGTAGTCAGTCAG-3' (SEQ ID NO. 20: P14) was used as the antisense primer. Using these PCR primers and using ExTaq (Takara Co., Ltd.) as the reaction enzyme, a cycle of 1 minute of heat denaturing at 94°C, followed by annealing and then 30 seconds of extension reaction at 72°C, was repeated 30 times (28 times in the case of KU-GB-3) using a thermal cycler (Perkin-Elmer). The annealing temperature was set to the Tm value of each primer [59°C for KU-GB-3, 62°C for KU-GB-4, 63°C for SOX5, 61°C for SOX6, 62°C for zinc finger homeobox, 62°C for M-phase phosphoprotein 1, and 68°C for β-actin, serving as the control] and was carried out for 30 seconds. The PCR products obtained were subject to agarose gel electrophoresis (2.0%), stained with ethidium bromide (EtBr), and then bands were detected by irradiation of ultraviolet rays of 254nm.

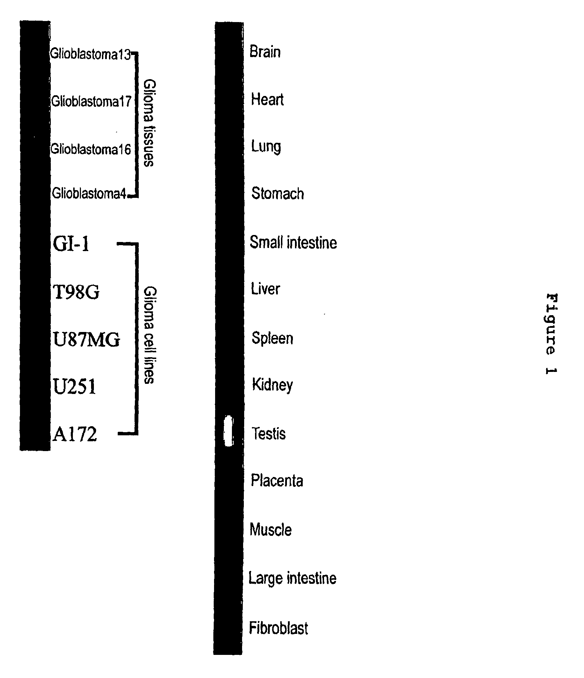

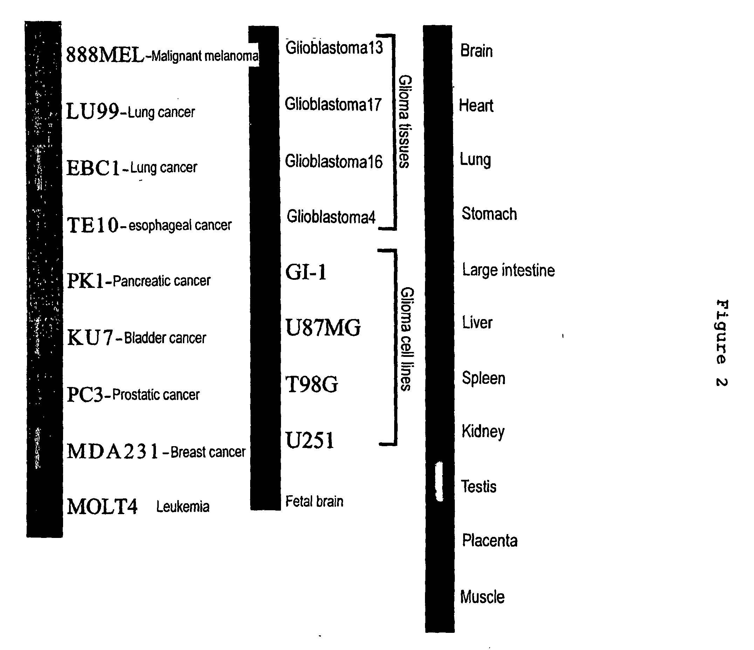

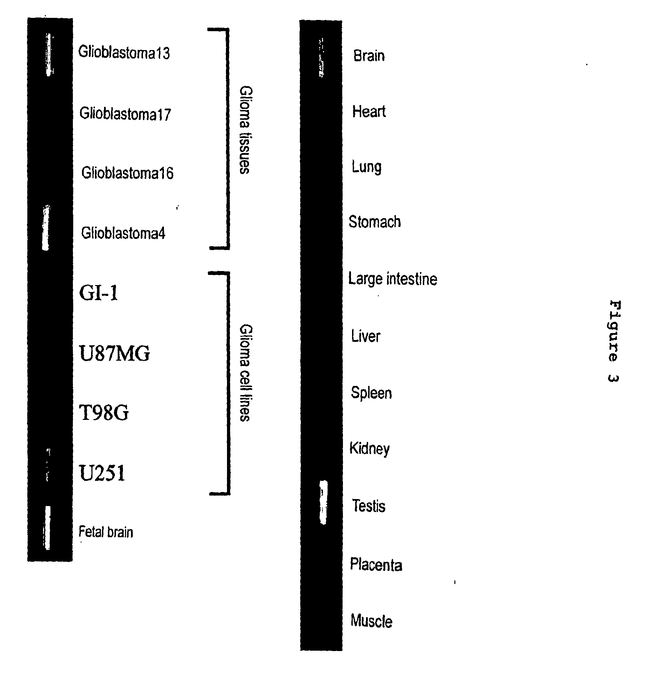

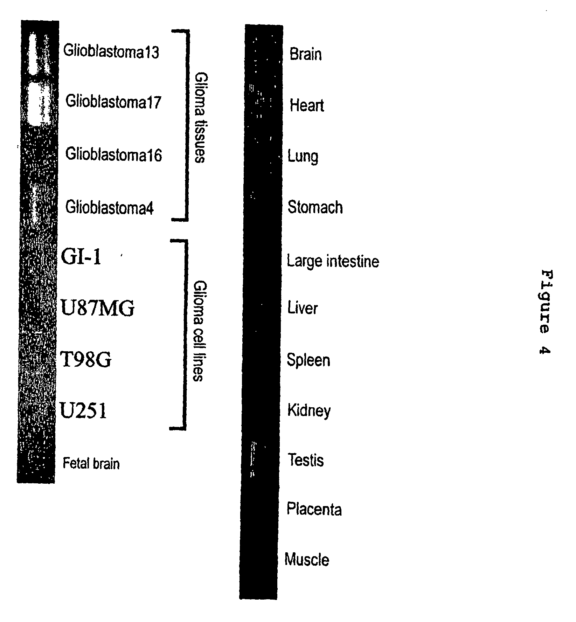

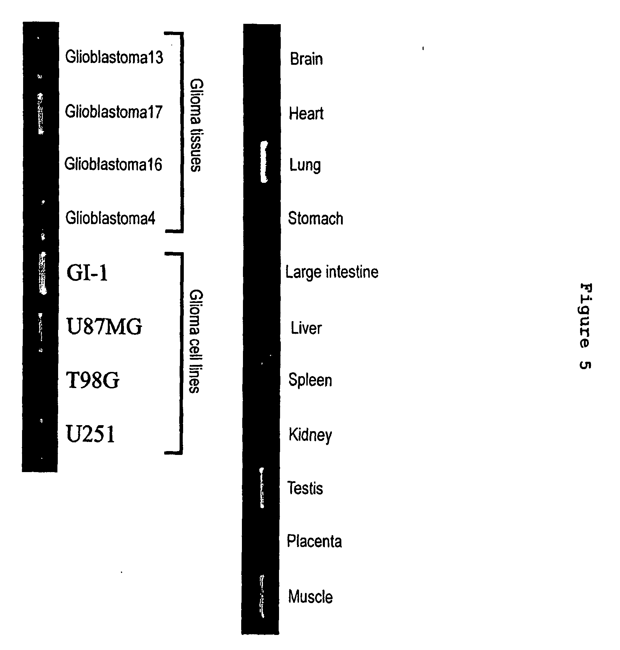

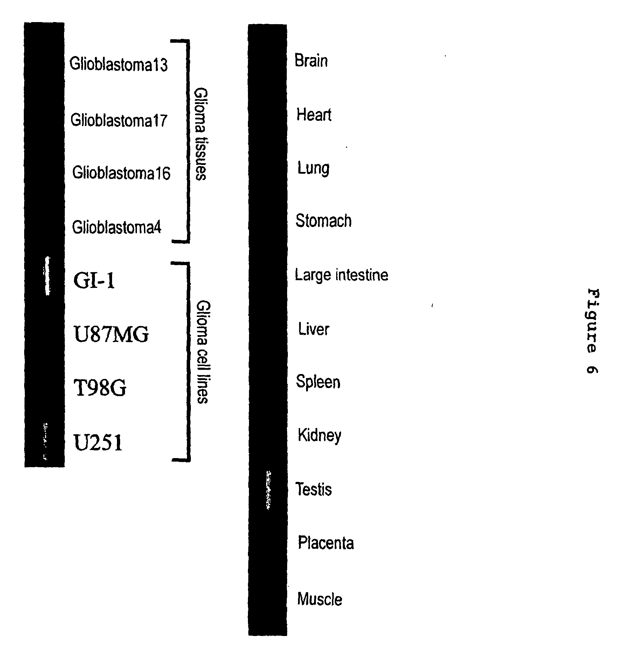

The results of the above are shown in Fig. 1 (KU-GB-3), Fig. 2 (KU-GB-4), Fig. 3 (SOX5), Fig. 4 (SOX6), Fig. 5 (zinc finger homeobox), and Fig. 6 (M-phase phosphoprotein 1), and basically all exhibited strong expression in testis. With the RT-PCR of KU-GB-3, expression was seen in lung, GB17, and GB16 and weak expression was seen in pancreas, GB4, and T98G (Fig. 1), and with the RT-PCR of KU-GB-4, weak expression was seen in LU99 (lung cancer cell line), EBC1 (lung cancer cell line), KU7 (bladder cancer cell line), PC3 (prostatic cancer cell line), MDA231 (breast cancer cell line), etc., (Fig. 2). With the RT-PCR of SOX5, strong expression was seen in GB13, GB4, and fetal brain, expression was seen in brain, GB16, and U251, and weak expression was seen in pancreas and GB17 (Fig. 3). With the RT-PCR of SOX6, strong expression was seen in GB13 and GB17 and weak expression was seen in GB4 (Fig. 4). With the RT-PCR of zinc finger homeobox, strong expression was seen in lung, GB17, and GI-1, expression was seen in muscle, GB13, GB16, GB4, U87MG, T98G, and U251, and weak expression was seen in pancreas, kidney, and placenta (Fig. 5). With the RT-PCR of M-phase phosphoprotein 1, strong expression was seen in GI-1 and U251 (Fig. 6).

The above results show that the genes for KU-GB-3, KU-GB-4, SOX5, SOX6, zinc finger homeobox, and M-phase phosphoprotein 1 are useful as genetic diagnostic drugs and treatment drugs for cancers, including glioma. In regard to expression in testis, since this tissue is isolated from the immune system, it is considered that there is hardly any possibility of testis disorders occurring when the KU-GB-3, KU-GB-4, SOX5, SOX6, zinc finger homeobox, or M-phase phosphoprotein 1 antigen is actually used in immunotherapy, etc.

### Example 2 (Characteristics of the testis-derived human glioma antigen SOX6)

The characteristics of SOX6, which was identified by SEREX as being a testis-derived human glioma antigen, were examined in more detail.

### [Preparation of recombinant SOX6 HMG box protein]

The cDNA of the SOX6 HMG box was subcloned in pET32a plasmids (Novagen) and the protein was expressed in the Escherichia coli, BL21 (DE3) Lys S (Novagen). The recombinant SOX6 was purified using the affinity His, HiTrap Chelating (Amersham Pharmacia).

### [Western blot analysis for evaluating the serologic reaction to the SOX6 HMG box]

The purified recombinant SOX6 HMG box was mixed with an equal volume of sodium dodecyl sulfate (SDS) - sample buffer, composed of 2% SDS, 10% β-mercaptoethanol, 10% glycerol, 1 mM EDTA, 40 mM Tris, and 240 mM glycine at pH 8.5, and boiled for 3 minutes. The same amount of total protein (10 µg) was loaded in respective lanes of SDS-polyacrylamide gels (10%). After electrophoresis, the gels were transferred to a nitrocellulose sheets (Hybond C⁺; Amersham Pharmacia) by electroblotting. After blocking with 5% skim milk for 2 hours, the sheets were incubated with 1:100 diluted sera from glioma patients for 1 hour at room temperature. After washing, the blots were incubated with 1:2000 diluted alkaline phosphatase-labeled goat antihuman IgG (Fc) antibody (Cappel Inc.). Enzymatic detection of bound secondary antibodies was then carried out using nitro blue tetrazolium (Boehringer Mannheim GmbH) and 5-bromo-4-chloro-3-indolyl phosphate (Sigma Chemical Co.). [ELISA for examining the serologic reaction to the SOX6 HMG box]

100 ng per well of the purified recombinant SOX6 HMG box was adsorbed to the wells of a 96-well plate overnight. Control wells were coated with purified recombinant protein pET 32a plasmid. After washing and blocking the wells with 2% BSA/PBS, 100µl of diluted human serum was added and incubation at 4°C was carried out for 1 hour. Examinations were carried out with the sera being diluted in the range of 1:100 to 1:50,000. The wells were washed and incubated for 1 hour with a peroxidase-conjugated antibody to human IgG, and this was followed by reaction with a TMB substrate (Dako) and measurement of the optical density at 450nm.

### [Quantitative RT-PCR and Northern blot]

Using SYBR green, which is a fluorescent dye, and the ABI Prism 7700 Sequence Detection System (Perkin-Elmer, Inc.), quantitative RT-PCR analysis was carried out by a method described in literature (Nat. Med. 4, 1329-1333, 1998). The thermal cycler parameters were set to 10 minutes at 95°C, 50 cycles of denaturation at 95°C for 30 seconds, annealing at 60°C for 1 minute, and extension at 72°C for 1 minute. The relative expression amount of SOX6 was computed by the following procedure. That is, the β-actin of each tissue was used for standardization and the (C_{T}) (threshold cycle value) of each sample was divided by the C_{T} value of normal brain. When the fluorescence signal increases to or above the background threshold level, the C_{T} value is defined as the number of actual PCR cycles and indicates the number of copies of the target gene.

For Northern blot analysis, 10 µg of total RNA was fractionated by electrophoresis in a 1% formaldehyde agarose gel and transferred to a nylon membrane (Hybond-XL: Amersham Pharmacia Inc.). Radioisotope-labeled cDNA fragments were prepared using the High Prime DNA Labeling Kit (Boehringer Mannheim GmbH). Prehybridization was performed at 65°C for 30 minutes using the Quick Hyb solution (Stratagene Inc.), and hybridization with the probes was carried out overnight at 65°C. The membrane was then washed twice for 15 minutes each at room temperature with 2 x SSC in 0.1% SDS, followed by an additional wash for 30 minutes at 60°C in 0.1 x SSC and 0.1% SDS. Radioactive signals were detected using the BAS5000 (Bio-Rad Inc.).

### [Transfection of SOX6 into 293T cells]

Full-length SOX6 cDNA was subcloned in the mammalian expression vector, pcDNA3myc (Invitrogen Inc.). The linearized cDNA was genetically introduced into a 293T cell line using Lipofectamine PLUS reagent (Life Technologies Inc.).

### [Isolation of nuclear extracts]

Normal brain tissue purchased from Clontech Laboratories Inc., glioma tissues, and 293T cells were homogenized in 0.25 M sucrose and centrifuged for 10 minutes. Pellets were resuspended in 0.25 M sucrose supplemented with a protease inhibitor cocktail (Sigma Aldrich Inc.).

### [Polyclonal antibodies]

A rabbit anti-SOX6 affinity purified polyclonal antibody, which recognizes a peptide (amino acid residues 349 to 364) of human SOX6, was purchased from Chemicon International, Inc.

### [Western blot and blocking analysis for confirming SOX6 expression]

SOX6 expressions in glioma and normal brain were examined by the above-described assay. 20 µg of total protein was loaded into each lane. The first antibody was the rabbit antihuman SOX6 affinity purified polyclonal antibody (10 µg/ml), and the second antibody was the alkaline phosphatase-labeled goat anti-rabbit IgG (Pc) antibody (Cappel Inc.) diluted by 1:2000. For the blocking experiment, the diluted SOX6 polyclonal antibody (10 µg/ml) was incubated with 293T cells expressing SOX6 at a final concentration of 20 µg/ml in 1% PBS and 0.5% skim milk, and then subject to Western blotting as described above.

### [Immunohistochemical staining]

Tumor samples and normal brain tissue were fixed in 10% formalin and then embedded in paraffin for immunohistochemical analysis. Sections (5 µm) were cut and mounted onto microscope slides coated with poly-L-lysine. The mounted tissue sections were deparaffinized with xylene and rehydrated. Antigen revival was carried out as described in literature (J. Pathol. 183, 116-123, 1997). Endogenous peroxidase was blocked by incubation with 0.3% hydrogen peroxide in methanol, followed by washing in phosphate-buffered saline (PBS). The nonspecific binding of antibodies was blocked by incubation in 0.02 M PBS, containing 5% BSA, and PBS, containing 0.1% Triton X-100, for 1 hour. The slides were then incubated overnight at 4°C with the antihuman SOX6 rabbit polyclonal antibody (1 µg/ml), which was diluted in the same blocking solution. The slides were incubated for 30 minutes at 37°C with a second antibody (Universal Immuno-peroxidase Polymer, Anti-Rabbit antibody; Histofine Simple Strain MAX PO (R), made by Nichirei Corporation), and the HRP labeling was visualized using DAB. The sections were lightly counterstained with hematoxylin. Each step was followed with three washes in PBS.

For evaluation of the proliferative activity of the tumors, sections were stained with the monoclonal antibody, MIB-1. Dewaxed and rehydrated sections in a 10mM citrate buffer of pH 7 were processed five times for 4 minutes each in a 600-watt microwave oven. After treatment for 1 hour at room temperature with 5% bovine serum albumin containing 3% horse serum, the sections were incubated overnight at 4°C with MIB-1 (diluted by 1:50, made by Immunotech Inc.). Primary antibodies were visualized by the avidin-biotin complex technique using DAB. Sections were lightly counterstained with hematoxylin. Proliferating cell indexes were analyzed using a total of 1,000 or more tumor cells at three or more regions expressing the highest numbers of immune-positive nuclei. The analysis was carried out using a computer-assisted image analyzer (Luzex F; Nireco Corp.).

### [Analysis of sera from healthy individuals, glioma patients, and patients with various cancers for examination of the presence of SOX6-specific IgG antibodies]

Serum screening by SEREX was carried out on various patients to confirm the specificity and relevance of the antibody response to SOX6. Sera from 36 glioma patients, 14 patients of other brain disorders, 54 patients with various cancers, and 37 healthy individuals ranging from 20 to 78 years in age were diluted by 1:100 and used to screen IgG antibodies specific to SOX6. Transcripts of SOX6 induced serological responses in 12 (33.3%) of the 36 glioma patients. However, transcripts of SOX6 induced serological responses in 0 (0%) of the 14 patients of other brain disorders and only 1 (1.9%) of the 54 patients with other cancers. With sera obtained from 37 normal adults, no antibody against the abovementioned protein was detected with the exception of the serum from one individual (Table 4).

**(Table 4)**

| Serum | Positive reaction |
|---|---|
| Glioma | 12/36 (33.3%) |
| Other brain disorders | 0/14 (0%) |
| Melanoma | 0/10 (0%) |
| Esophageal cancer | 0/10 (0%) |
| Pancreatic cancer | 1/10 (10%) |
| Bladder cancer | 0/10 (0%) |
| Colorectal cancer | 0/ 7 (0%) |
| Renal cell cancer | 0/ 7 (0%) |
| Healthy individuals | 1/37 (2.7%) |

### [The immunogenic epitope of the SOX6 protein]

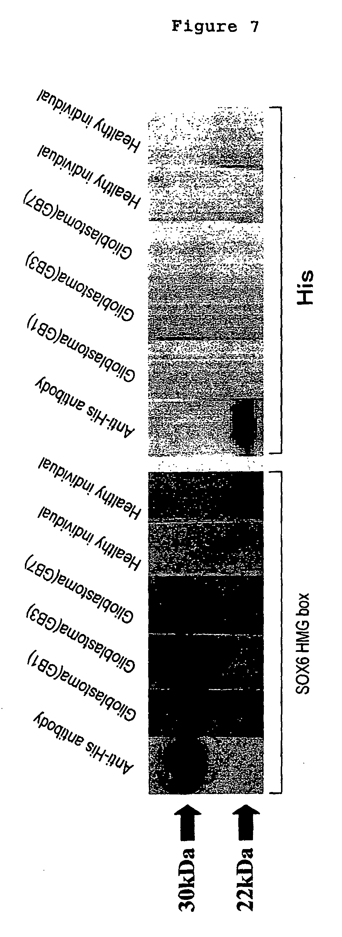

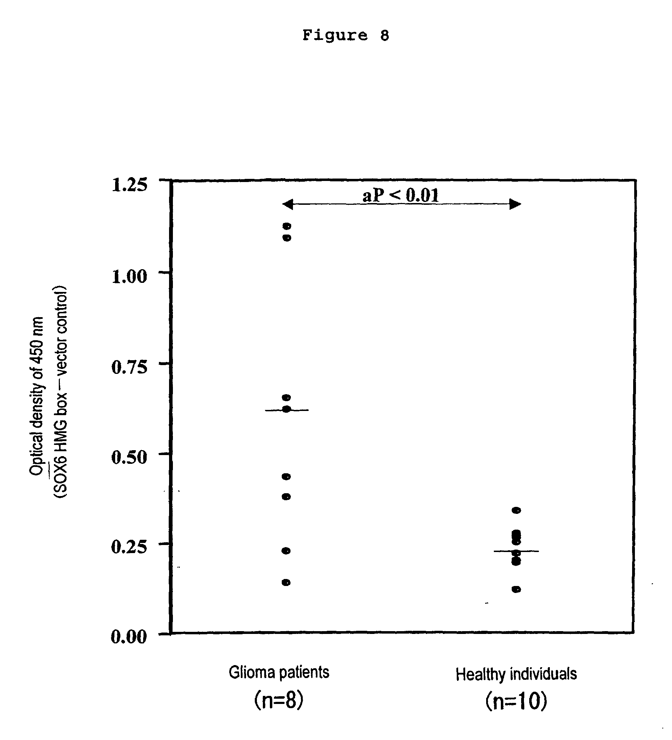

Since it has been suggested that the conserved HMG box domain, which is a DNA binding site of SOX6, may be recognized by IgG derived from glioma patient sera, the sequence encoding the HMG box of SOX6 was subcloned into the pET32a vector to prepare a His-tagged recombinant protein. First, the seroreactivity against the HMG box of SOX6 was examined by Western blot analysis. The IgG in sera obtained from three of eight glioma patients, who were found by SEREX to exhibit positive seroreactivity against SOX6, detected a 30 kDa protein corresponding to the HMG box expressed by recombination (Fig. 7). To confirm the predicted immunogenic epitope more quantitatively, the serologic reactivity to the SOX6 HMG box protein was examined by ELISA. As a result, little serologic reactivity to His was observed among healthy individuals and among glioma patients having antibodies to SOX6. On the other hand, with regard to the reactivity of serum antibodies to the SOX6 HMG box a significant difference was observed between healthy individuals and the abovementioned glioma patients, as was observed in the Western blot analysis results (Fig. 8).

### [Analysis of SOX6 gene expression in glioma and normal tissues by RT-PCR and Northern blotting]

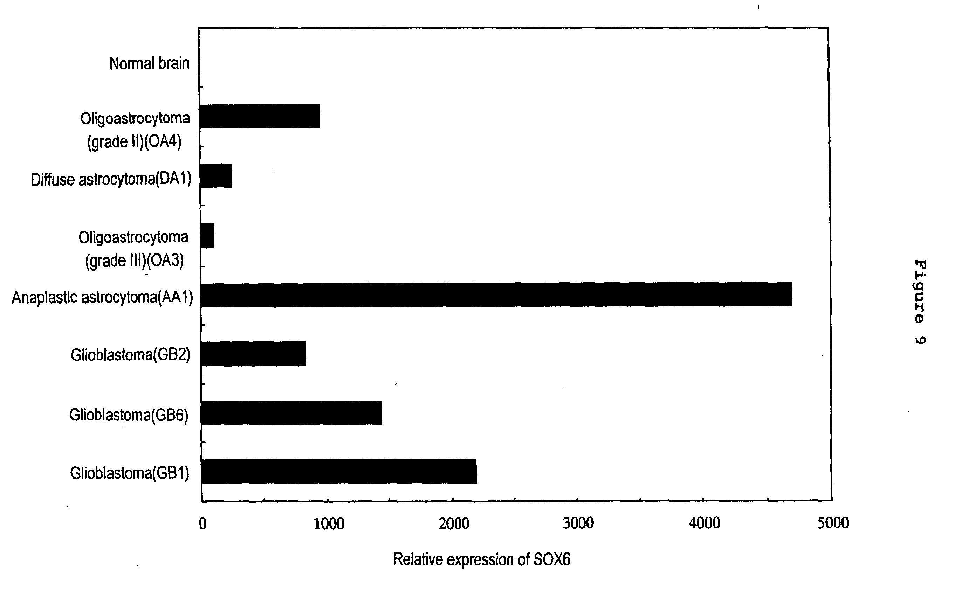

In order to examine the SOX6 gene expression amounts in glioma and normal adult tissues, the differences of SOX6 gene expression between glioma and normal adult brains were evaluated by quantitative RT-PCR using SYBR Green. In comparison to a normal adult brain, SOX6 was expressed in significantly large amounts in all glioma tissues that were analyzed (Fig. 9).

It has been reported that two transcripts, one long and one short, are detected from the mouse SOX6 gene (Proc. Natl. Acad. Sci. USA, 97, 4180-4185, 2000; Embo. J. 17, 5718-5733, 1998) and the human SOX6 gene (Gene 265, 157-164, 2001). The difference in the lengths of these transcripts is most likely caused by the different sizes of both the 3' and 5' untranslated regions (Embo. J. 17, 5718-5733, 1998). The short transcript is specifically expressed in adult testis, and the long transcript is expressed in mouse brain (Nucleic Acids Res. 23, 3365-3372, 1995) and chondrocytes (Embo. J. 17, 5718-5733, 1998) during development but disappear from these tissues in adults.

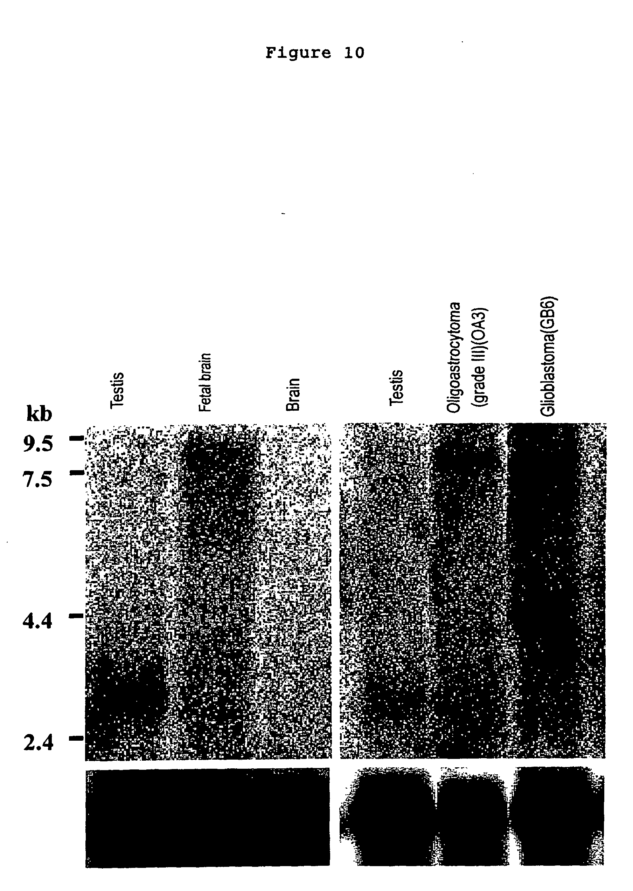

To investigate which transcript is expressed in glioma, the expression of the SOX6 mRNA in human glioma was determined by Northern blotting (Fig. 10). Though the long SOX6 transcript was expressed in human fetal brain, it was not expressed in adult brain. The short transcript was expressed mainly in testis. In glioma tissues, the long transcript was expressed in extremely large amounts. In conclusion, the SOX6 gene is expressed in the early stages of human neural development and is expressed selectively in adult testis and glioma.

### [Selective expression of the SOX6 protein in glioma]

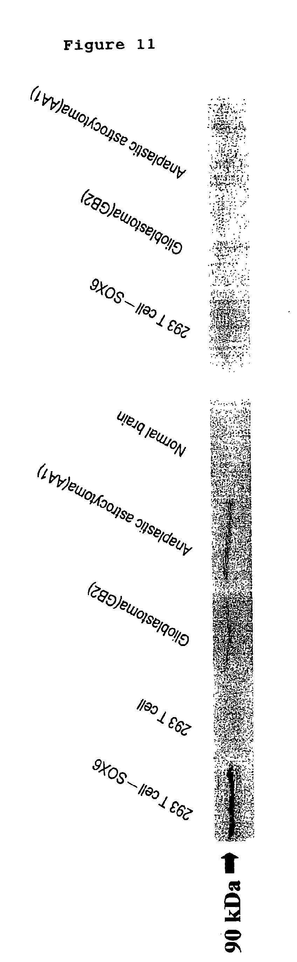

The expression of the SOX6 protein was evaluated by Western blotting using a polyclonal antibody. The antibody detected the 90 kDa band of the SOX6 protein in 293T cells transfected with the SOX6 cDNA. On the other hand, the antibody did not detect the same band in 293T cells that were not transfected by the SOX6 cDNA (left side of Fig. 11). Though the SOX6 protein was expressed in glioma, it was not expressed in normal brain tissue. Such selective expression of the SOX6 protein in glioma agrees with the results obtained by quantitative RT-PCR and Northern blotting.

The specificity of the abovementioned polyclonal antibody was confirmed by incubating the 293T cells that express SOX6 with the antibody. The 90 kDa signal in the immunoblot disappeared in this blocking experiment using the polyclonal antibody (right side of Fig. 11), and this shows that the abovementioned antibody recognizes SOX6 specifically.

### [Immunohistochemical analysis of SOX6 in glioma]

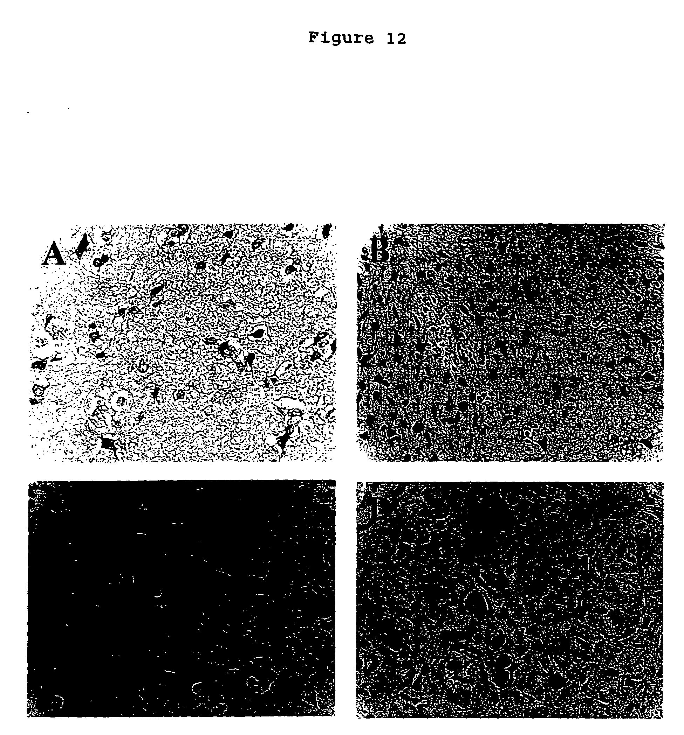

Using the antibody against SOX6, sectioned materials from formalin-fixed tumors were analyzed, and the proliferative activity was evaluated using the MIB antigen. All of the analyzed eighteen types of glioma tissues expressed SOX6 in nuclear tumor cells. On the other hand, SOX6-positive cells were not detected from nonneoplastic tissue derived from the cerebral cortex (Fig. 12 and Table 5). Fig. 12 shows the immunohistochemical analysis results of SOX6 expression in nonneoplastic tissue derived from the cerebral cortex (Fig. 12A) and the respective tissues of diffuse astrocytoma (case OA4; Fig. 12B), anaplastic astrocytoma (case AA1; Fig. 12C), and glioblastoma (case GB4; Fig. 12D).

The correlation of SOX6 expression with histological malignancy, proliferation, and seroreactivity in glioma are summarized in Table 5. SOX6 expression was confirmed in all tumor tissues from patients having IgG antibodies against SOX6. Furthermore, though the possibility of a relationship between the expression of SOX6 and proliferative activity in glioma was evaluated, a clear correlation was not seen between the two.

**(Table 5)**

| Case No. | Histology | Age | Sex | SEREX^{a} | MIB-1^{b} | Immunohistochemistry^{c} |
|---|---|---|---|---|---|---|
| <WHO grade IV> | | | | | | |
| GB1 | Glioblastoma | 25 | F | + | 41.1 | +++ |
| GB2 | Glioblastoma | 31 | M | + | 12.7 | +++ |
| GB3 | Glioblastoma | 7 | M | + | 17.7 | +++ |
| GB4 | Glioblastoma | 50 | M | NA | 14.5 | +++ |
| GB5 | Glioblastoma | 34 | F | + | 0.9 | +++ |
| GB6 | Glioblastoma | 41 | F | + | 0.8 | +++ |
| GB7 | Glioblastoma | 21 | F | + | 2.1 | ++ |
| GB8 | Glioblastoma | 14 | M | + | 28.1 | + |

| <WHO grade III> | | | | | | |
|---|---|---|---|---|---|---|
| AA1 | Anaplastic astrocytoma | 28 | M | + | 4.5 | +++ |
| AA2 | Anaplastic astrocytoma | 26 | M | - | 5.2 | ++ |
| AA3 | Anaplastic astrocytoma | 36 | M | - | 1.2 | ++ |
| AA4 | Anaplastic astrocytoma | 30 | F | + | 2.3 | + |
| AA5 | Anaplastic astrocytoma | 37 | F | + | 1.5 | + |
| AA6 | Anaplastic astrocytoma | 32 | F | + | NA | NA |
| OA1 | Oligoastrocytoma (grade III) | 31 | M | - | 15.1 | +++ |
| OA2 | Oligoastrocytoma (grade III) | 41 | M | - | 5.2 | +++ |
| OA3 | Oligoastrocytoma (grade III) | 51 | M | - | 6.8 | + |

| <WHO grade II> | | | | | | |
|---|---|---|---|---|---|---|
| DA1 | Diffuse astrocytoma | 3 | F | - | 2.7 | + |
| OA4 | Oligoastrocytoma (grade II) | 7 | F | + | NA | ++ |

| | | | | | | |
|---|---|---|---|---|---|---|
| a The seroreactivities of SOX6 as determined by SEREX are indicated as +: positive; -: negative. | | | | | | |
| b The proliferating cell indexes were analyzed in a total of 1000 or more tumor cells in three or more regions expressing the highest numbers of MIB-1 positive nuclei. | | | | | | |
| c The staining patterns are described as -: negative or faint staining; +: positive in less than 30% of tumor cells; ++: positive in 30% to less than 70% of tumor cells; +++: positive in 70% or more of tumor cells. NA: not available; M: male; F: female. | | | | | | |

The above Example clearly shows SOX6 to be a new glioma expression antigen that causes an immunological response in no less than 33% of glioma patients. The high level of immunological response to the SOX6 protein is considered to be due to high expression of the SOX6 gene. Furthermore, though the expression of SOX6 in normal tissue is limited, it is expressed primarily in glioma tissue. The above results show that SOX6 can be a useful target for the development of diagnostic means and treatments for glioma.

### Industrial Applicability

The present invention's testis-derived glioma antigens and/or glioma antigen genes are useful for the treatment and diagnosis of glioma and other cancers and are also useful for obtaining fundamental knowledge concerning gliomagenesis.

## Claims

1. A DNA encoding a protein comprising the amino acid sequence indicated by SEQ ID NO. 2 or 4, or a DNA encoding a protein comprising an amino acid sequence in which one or several amino acids have been deleted, substituted, or added with respect to the amino acid sequence indicated by SEQ ID NO. 2 or 4 and having an immunity induction activity.

2. A DNA comprising the base sequence indicated by SEQ ID NO. 1 or 3 or a base sequence complementary to the same or a base sequence containing a part or the whole of the same.

3. A DNA which hybridizes under stringent conditions with the DNA according to claim 2 and encodes a protein having an immunity induction activity.

4. An oligonucleotide or peptide nucleic acid which hybridizes specifically with the DNA according to claim 3 or with an RNA, corresponding to this DNA, and inhibits the expression of a protein having an immunity induction activity.

5. A protein comprising the amino acid sequence indicated by SEQ ID NO. 2 or 4, or a protein comprising an amino acid sequence in which one or several amino acids have been deleted, substituted, or added with respect to the amino acid sequence indicated by SEQ ID NO. 2 or 4 and having an immunity induction activity.

6. A peptide comprising a part of the protein according to claim 5 and having an immunity induction activity.

7. A fusion protein or fusion peptide, wherein the protein according to claim 5 or the peptide according to claim 6 is bound with a marker protein and/or a peptide tag.

8. An antibody against the protein according to claim 5 or the peptide according to claim 6.

9. A recombinant vector containing the DNA according to any of claims 1 to 4.

10. A host cell containing an expression system that can express the protein according to claim 5 or the peptide according to claim 6.

11. A non-human animal, in which a genetic function encoding the protein according to claim 5 or the peptide according to claim 6 is absent from the chromosomes or which overexpresses the protein according to claim 5 or the peptide according to claim 6.

12. An immunity induction activity promoter or inhibitor screening method using the protein according to claim 5 or the peptide according to claim 6, a test substance, and T cells and measuring and evaluating the immunity induction activity in the T cells.

13. A testis-derived antigenic protein having an immunity induction activity comparable to a glioma antigenic protein and being obtained by the steps of:
(1) extracting and purifying total RNA from testis tissue, synthesizing cDNA using the purified mRNA, and constructing a λ phage cDNA library by introducing the cDNA into a λ phage vector and infecting Escherichia coli with the vector;
(2) diluting the serum of a glioma patient, reacting it with an extract of the Escherichia coli, and removing the reaction products to prepare a reaction serum;
(3) reacting the abovementioned λ phage cDNA library with the reaction serum and using a labeled anti-IgG antibody to detect positive clones that react with the antibody in the serum;
(4) repeating the screening process several times on the detected positive clones to confirm the antibody reactivity; and
(5) isolating an antigen from the positive clones and performing serum screening using the isolated antigen and at least a glioma patient serum and a serum of a healthy individual.

14. The testis-derived antigenic protein according to claim 13, comprising KU-GB-4, KU-GB-3, a SOX family molecule, zinc finger homeobox (zinc finger homeobox 1B), an ADP-ribosylation factor (ADP-ribosylation factor 4-like), or M-phase phosphoprotein 1.

15. The testis-derived antigenic protein according to claim 14, wherein the SOX family molecule is SOX5, SOX6, or SOX13.

16. A glioma detection diagnostic drug containing the whole or a part of the protein according to any of claims 13 to 15 and/or an antibody binding specifically with the whole or a part of the protein according to any of claims 13 to 15.

17. A glioma diagnosis method **characterized in** using an antibody binding specifically with the whole or a part of the protein according to any of claims 13 to 15.

18. The glioma diagnosis method according to claim 17, wherein a test sample is a serum and ELISA or Western blotting is employed.

19. The glioma diagnosis method according to claim 17, wherein a test sample is a cell tissue and an immunohistochemical analysis method is employed.

20. A glioma detection/diagnosis probe containing the whole or a part of an antisense chain of a DNA or RNA encoding the protein according to any of claims 13 to 15.

21. An antitumor agent **characterized in** containing the whole or part of the protein according to any of claims 13 to 15 and/or an antibody binding specifically with the whole or part of the abovementioned glioma antigen as an effective component.

22. A tumor prevention/treatment drug using the antitumor agent according to claim 21.

23. A glioma recognizing antibody specifically recognizing glioma in immunohistochemical analysis.

24. The glioma recognizing antibody according to claim 23, which is an anti-SOX6 antibody.

25. The glioma recognizing antibody according to claim 24, wherein the anti-SOX6 antibody specifically recognizes the HMG box of SOX6.

26. A glioma detection diagnostic drug containing the glioma recognizing antibody according to any of claims 23 to 25.

27. A glioma diagnosis method using the glioma recognizing antibody according to any of claims 23 to 25.

28. The glioma diagnosis method according to claim 27, wherein a test sample is a serum and ELISA or Western blotting is employed.

29. The glioma diagnosis method according to claim 27, wherein a test sample is a cell tissue and an immunohistochemical analysis method is employed.

30. A glioma diagnosis method using the glioma recognizing antibody according to any of claims 23 to 25.
